# FASCICULE DE BREVET EUROPEEN

(11) **EP 3 475 406 B1**
(45) Date de publication et mention de la délivrance du brevet: **15.03.2023**
(21) Numéro de dépôt: 17745796.7
(22) Date de dépôt: 26.06.2017
(51) Int. Cl.: C12M 1/00, C12M 3/06, F16K 7/06

(54) **CASSETTE ET AUTOMATE DE CULTURE CELLULAIRE**
KASSETTE UND AUTOMAT FÜR ZELLKULTUR
CASSETTE AND AUTOMATON FOR CELL CULTURE

(30) Priorité: 24.06.2016 FR 1655922
(43) Date de publication de la demande: 01.05.2019
(73) Titulaire: CellProthera, 68100 Mulhouse (FR)
(72) Inventeur: VALAT, Christophe, 68100 Mulhouse (FR); HENON, Philippe, 68100 Mulhouse (FR); SAUCOURT, Claire, 68100 Mulhouse (FR); WEIL, Raoul, 67120 Dorlisheim (FR); SERRE, Jérôme, 84120 Pertuis (FR); MARECHAL, Cyrille, 63160 Billom (FR)
(74) Mandataire: Ernest Gutmann - Yves Plasseraud S.A.S.
(86) Numéro de dépôt international: PCT/FR2017/051703
(87) Numéro de publication internationale: WO 2017/220948

(56) Documents cités:
- DE-A1-102014 105 472
- US-A- 6 096 532
- US-A1- 2013 058 907
- US-A1- 2013 244 322

## Description

La présente invention se rapporte à une cassette de culture cellulaire, ainsi qu'à un automate de culture cellulaire utilisant ladite cassette. L'invention porte ainsi ici sur un dispositif pour la culture cellulaire, notamment, désigné sous le nom de cassette.

Parmi les domaines faisant appel à la culture cellulaire, celui de la thérapie cellulaire est le moins avancé en termes d'industrialisation. Il existe donc un besoin important de trouver une technologie capable de produire des cellules en quantité suffisante et dans des conditions optimales et sécurisées en vue de l'utilisation de ces cellules pour des applications thérapeutiques.

Certains procédés de thérapie cellulaire nécessitent une culture ou amplification de cellules souches avant leur réinjection chez un patient, car les quantités prélevées sont parfois insuffisantes pour avoir un effet thérapeutique. Il est indispensable de garantir l'intégrité des propriétés thérapeutiques des cellules pendant leur culture. Dans la technique actuelle, les solutions proposées pour cultiver les cellules souches *ex vivo* sont artisanales, très empiriques et peu efficaces.

De plus, la technique actuelle ne permet pas de produire des cellules souches en quantité suffisante pour des applications thérapeutiques. Il existe donc un réel besoin de développer une technologie du type bioréacteur ayant une géométrie compacte et permettant de cultiver des cellules en grande quantité.

L'automation des procédés d'expansion de cellules souches pour des applications de production dans un cadre thérapeutique ou clinique nécessite des dispositifs de production de cellules capables de fonctionner dans des zones d'activités contrôlées (salle blanche), en réduisant au maximum l'intervention humaine. Les procédés de culture de cellules souches sont relativement complexes car ils mettent en oeuvre de multiples étapes d'obtention de ces cellules à partir d'un prélèvement issu d'un patient, d'isolement des cellules, de purification avant mise en culture et enfin de collecte, de purification et de conditionnement après culture avant envoi vers les centres de traitements cliniques.

L'ensemble de ces étapes doit se faire dans le respect de normes de fabrication imposant des conditions de stérilité et de traçabilité strictes. La multiplication des étapes de fabrication, souvent manuelles nécessitent l'utilisation de zones de confinement de classes différentes afin de maintenir la stérilité du procédé. La bonne conduite du procédé de préparation avant et après la culture implique souvent le transfert des produits intermédiaires du procédé d'une zone à une autre, augmentant à la fois les risques de perte de stérilité et de mélange des flux de production.

On connait par exemple un système de culture cellulaire tel que celui décrit dans le document US2012/0308531. La réalisation de cultures en continue destinées à la production de cellules souches s'effectue au moyen d'un système complexe de sous-ensembles amenant les différents réactifs, cellules et gaz au réacteur.

La demanderesse a également proposé un automate de culture cellulaire automatisé dans le document WO/2013/135817. Si la culture cellulaire est effectuée au sein d'une seule et même poche, la manipulation de celle-ci peut s'avérer délicate du fait de sa structure souple.

L'invention a notamment pour but d'apporter une solution simple, efficace et économique aux problèmes de l'art antérieur décrit précédemment.

A cet effet, elle propose une cassette de culture cellulaire comprenant :
- un boîtier au moins partiellement rigide et définissant intérieurement un espace intérieur dans lequel est agencée et fixée une poche de culture cellulaire définissant un volume intérieur,
- des conduits reliés chacun à une extrémité au volume intérieur de la poche et ayant chacun une seconde extrémité située à l'extérieur du boîtier, et
- des vannes d'ouverture/fermeture de la circulation de fluide au travers des conduits qui sont montées sur le boîtier.

La manipulation de la poche et des vannes s'effectue au travers du boîtier de la cassette. Seules les secondes extrémités des conduits nécessitent une manipulation directe. Selon l'invention, la cassette ou dispositif de culture cellulaire intègre tous les éléments susceptibles de poser une difficulté en termes de stérilité et dont la manipulation directe doit être limitée. Ces éléments sont préassemblés dans une cassette de culture cellulaire à usage unique. Après utilisation, il est possible d'éliminer selon les règles de bonnes pratiques l'ensemble des conduits, vannes et poche de culture utilisés. De préférence, l'intégration d'une poche souple dans un boîtier au moins partiellement rigide facilite grandement la manipulation de la poche qui est ainsi retenue dans le boîtier. De même, l'intégration des vannes dans le boîtier permet de faciliter le repérage des positions des vannes qui peuvent dès lors être plus aisément manipulées par des moyens de commande d'un incubateur comme cela apparaitra dans la suite de la description.

La solution d'intégration des vannes dans la cassette fluidique comme moyen d'occlusion des conduits de la cassette permet d'améliorer l'automatisation de l'utilisation de la cassette dans un procédé de culture cellulaire en évitant l'utilisation d'une part des « clamps » en plastique, souvent fragilisés lors d'une étape de congélation, et manoeuvrables uniquement à la main, ou d'autre part d'électrovannes à pincement nécessitant la mise en place manuelle des conduits dans la mâchoire de l'électrovanne. Cette solution permet donc d'éviter la manipulation des conduits, et les erreurs liées à la mauvaise insertion des conduits dans les électrovannes.

La cassette au sein de laquelle la poche comprend un milieu de culture cellulaire peut être congelée aussi longtemps que nécessaire.

Selon une autre caractéristique de l'invention, les conduits sont formés par des tubulures souples et au moins certaines des vannes comprennent un organe mobile de pincement de la tubulure sur un élément de structure du boîtier. Comme décrit précédemment, l'intégration des vannes dans la casette permet de garder de façon autonome et sécurisée les voies fermées (temps de stockage ou culture) pendant un temps voulu sans que cela ne nécessite d'énergie extérieure.

Selon encore une autre caractéristique de l'invention, chaque organe mobile comprend un axe de rotation et la périphérie radialement externe de chaque organe mobile comprend au moins une surface en spirale autour de l'axe de rotation formant une surface de pincement de la tubulure de manière à faire varier une section de passage de fluide au travers d'une tubulure au fur et à mesure de la rotation de l'organe.

Avantageusement, les vannes sont aptes à conserver une position donnée. Plus spécifiquement, les vannes sont conformées de manière à ce que les organes mobiles puissent conserver une position donnée d'ouverture ou de fermeture en l'absence d'apport d'énergie. Encore plus particulièrement, cela peut être obtenu en faisant en sorte que la perpendiculaire au point de contact de la surface en spirale avec une tubulure soit orientée vers l'axe de rotation de l'organe mobile, ce qui induit un couple nul autour dudit axe.

Dans une réalisation particulière, les organes mobiles sont montés dans des logements d'une platine solidaire du boîtier, chaque logement recevant un organe mobile. Lorsque le boitier est réalisé en deux demi-coque inférieure et supérieure, il est ainsi possible de monter la poche de culture cellulaire sur la demi-coque inférieure, de monter les organes mobiles de pincement des tubulures sur la demi-coque inférieure puis de monter la platine qui assure un maintien simultané des organes mobiles. La platine peut être, par exemple, solidarisée par exemple par vissage, à la demi-coque inférieure. La platine permet ainsi de sécuriser et de faciliter l'assemblage des différentes pièces précitées. Elle permet de garantir le maintien des vannes.

Chaque organe mobile comprend une extrémité accessible depuis l'extérieur du boîtier et pourvue de premiers moyens d'accouplement en rotation destinés à coopérer avec des seconds moyens d'accouplement en rotation d'un actuateur qui peut être électromécanique.

L'accès aux organes mobiles des vannes depuis l'extérieur rend possible le montage de la cassette sur un support adéquat d'un incubateur comportant des seconds moyens d'accouplement par exemple complémentaires pour l'entrainement en rotation des premiers moyens d'accouplement des vannes pour réaliser une ouverture/fermeture de la circulation de liquide selon les besoins. Cette accessibilité externe rend possible la manipulation manuelle des vannes en cas de défaillance des moyens d'entrainement des vannes.

Préférentiellement, les organes mobiles sont centrés et guidés à rotation à une extrémité dans un orifice du boîtier et à une extrémité opposée dans un orifice de la platine. Lorsque le boîtier est formé de deux demi-coques, la platine peut être serrée à l'intérieur du boîtier entre ces deux demi-coques.

Selon une autre caractéristique de l'invention, la poche présente une forme sensiblement parallélépipédique comportant une première paroi souple et une seconde paroi souple sensiblement parallèles l'une à l'autre à l'état vide et à l'état remplit de liquide.

La forme parallélépipédique est sensiblement plane de sorte que l'épaisseur mesurée entre la première et la seconde parois, dans une première direction sensiblement perpendiculaire aux premières et secondes parois, est très faible, c'est-à-dire très nettement inférieure aux deux autres dimensions de la poche mesurées dans les deux autres directions de l'espace perpendiculaires l'une à l'autre et à ladite première direction. On entend par « très nettement inférieur » au moins 10 fois inférieur comme il est communément admis en mathématique.

De préférence, le rapport de la somme des surfaces de la première paroi et de la seconde paroi sur le volume interne total de la poche est compris entre 500 et 690 cm²/L, de préférence est de l'ordre de 580 cm²/L. Ce ratio assure un échange de chaleur optimal entre la poche flexible et l'extérieur lorsque la cassette est agencée dans un incubateur à enceinte thermostatée.

La distance séparant la première paroi de la seconde paroi ou épaisseur de la poche à l'état remplit de liquide, mesurée selon une direction perpendiculaire aux première et seconde parois, est avantageusement inférieure à 20 mm, de préférence compris entre 10 et 20 mm et encore plus préférentiellement de l'ordre de 14 mm. L'utilisation d'une faible épaisseur permet encore de faciliter la diffusion de chaleur au sein du liquide de la poche. Cela permet également une congélation et une décongélation uniforme des différents constituants du milieu de culture, qui plus est rapide à température constante.

La poche utilisée dans la cassette selon l'invention permet de maintenir la stabilité du milieu de culture lors d'une étape de congélation par surgélation. En effet, une vitesse de congélation élevée des produits biologiques dérivés du sang est une garantie de préservation des constituants, d'homogénéité du milieu après décongélation, et d'absence d'effet de concentration des sels et des protéines en solution au coeur de la masse congelée. La poche selon l'invention favorise une surgélation rapide du milieu de culture grâce à sa géométrie particulière ayant un rapport surface / volume élevé, et une faible épaisseur.

Selon une autre caractéristique de l'invention, les parois de la poche sont perméables aux gaz, en particulier au CO2, et ont de préférence des propriétés limitant au maximum l'adhérence des cellules avec les parois de la poche.

La poche est de préférence souple, c'est-à-dire déformable. Elle comprend, de préférence, des parois souples perméables aux gaz et aptes à retenir le milieu de culture et son expansion cellulaire qui est liquide. En pratique, il s'agit de retenir tout liquide contenu dans la poche. Elle présente de préférence une bonne perméabilité à l'oxygène et au dioxyde de carbone, ce qui permet une bonne aération du contenu de la poche sans ouverture de celle-ci et donc sans risque de contamination de son contenu. Dans une réalisation particulière de l'invention, la poche comprend les caractéristiques suivantes de perméabilité (en cm³ par jour sous une pression d'une atmosphère, c'est-à-dire 1 bar, à 37°C) : pour le dioxygène 418 à 508, pour le dioxyde de carbone 699 à 1200, pour le diazote 157 à 200 et pour l'eau 0,05 à 0,1.

La poche est par exemple formée d'un film mince qui peut être réalisé en copolymère tel que du fluoropolymère et plus particulièrement du fluoro-éthylène-propylène pouvant avoir une épaisseur par exemple de l'ordre 120µm. Dans une réalisation préférée, la poche a une forme sensiblement parallélépipédique de faible épaisseur pouvant atteindre une largeur de 230 à 250 mm maximum à plat et une longueur de 230 à 250 mm à plat.

L'utilisation d'un matériau appartenant à la famille des fluoropolymères présente l'avantage d'être perméable aux gaz et également de présenter une faible adhérence par rapport aux liquides.

Le boîtier présente de préférence une forme correspondant sensiblement à celle d'un parallélépipède rectangle et peut comporter une demi-coque inférieure et une demi-coque supérieure solidaires l'une de l'autre, la demie-coque inférieure étant rigide et la demi-coque supérieure étant rigide ou flexible.

Ainsi, le boîtier peut présenter une forme également sensiblement plane présentant une très faible épaisseur en comparaison de sa longueur et de sa largeur.

Le boîtier peut être réalisé dans tout matériau thermoplastique élastomère tels que du SEBS désignant du Styrène-Ethylène-Butadiène-Styrène. La demi-coque inférieure peut être rigide pour assurer un maintien optimal de la poche flexible tandis que la demi-coque supérieure peut être partiellement rigide et comporter une ou plusieurs zones flexibles réalisées dans un matériau élastomère de manière à faciliter la préhension et une certaine déformabilité pour faciliter le montage de la cassette dans les moyens de support et d'agitation qui sera présenté ci-après. Bien évidemment, la demi-coque supérieure peut également être totalement rigide comme la demi-coque inférieure.

Les deux demi-coques inférieure et supérieure peuvent être assemblées par clipsage, collage ou soudure de leurs rebords périphériques. Les demi-coques peuvent avoir la même épaisseur ou des épaisseurs différentes.

Dans une réalisation préférée de l'invention, la demi-coque supérieure peut comprendre une ouverture ou évidement central dont la forme et la dimension sont déterminées pour permettre la propagation d'une ondulation du liquide de la poche dans l'ouverture lorsque la cassette contenant un liquide subit une oscillation autour d'un axe horizontal, l'ouverture étant disposée vers le haut. La formation d'une ondulation est souhaitable pour réaliser une homogénéisation rapide du liquide de la poche. La formation d'un évidement, qui pourrait encore être qualifié de découpe ou d'ajour, évite ainsi d'avoir à augmenter l'épaisseur du boîtier. Cet évidement assure également une meilleure diffusion des gaz au contact de la poche. Il peut avoir une dimension d'environ 210 mm de large et de l'ordre de 28 mm de long.

Préférentiellement, la demi-coque inférieure comprend une pluralité d'orifices, ayant de préférence un diamètre par exemple inférieur à 20 mm. L'intégration d'orifices permet de faciliter le transfert de gaz de manière similaire à l'ouverture de la demi-coque supérieure mais assure également un maintien de la poche sur la demi-coque inférieure.

Dans une réalisation pratique de l'invention, la demi-coque inférieure peut comprendre environ 50 et 400 orifices ayant chacun un diamètre compris entre 5 et 20 mm, par exemple de l'ordre de 10 mm. Les orifices peuvent être répartis selon un maillage ayant un motif de base qui est celui d'un triangle équilatéral de longueur unitaire comprise entre 5 et 40 mm et par exemple de 20 mm.

Dans une réalisation de la cassette selon l'invention, les conduits s'étendent au travers de l'un des rebords périphériques de l'une des demi-coques inférieure ou supérieure. Les conduits peuvent être logés dans des encoches de l'un des rebords périphériques de l'une demi-coques inférieure ou supérieure.

La cassette comprend, de préférence, une barrette de support des conduits, agencée à l'extérieur du boîtier et traversée par au moins certains des conduits. Cette barrette permet le support des conduits qui s'étendent à l'extérieur du boîtier de la cassette, ce qui limite les mouvements intempestifs des extrémités libres des conduits. De plus, le support est destiné à former un élément de passage étanche de la porte d'un incubateur comme cela apparaitra mieux dans la suite de la description.

La cassette comprend de préférence un milieu de culture cellulaire stocké dans la poche, le milieu de culture cellulaire pouvant être à l'état congelé.

Le milieu de culture cellulaire peut être un milieu de culture cellulaire dont la composition est adaptée à la multiplication de cellules de mammifère, en particulier de cellules humaines, à l'exclusion des cellules souches embryonnaires humaines. Le milieu de culture peut comprendre, ou ne pas comprendre, de cellules en culture.

Ces cellules, en particulier ces cellules humaines, à l'exclusion des cellules souches embryonnaires humaines, peuvent avantageusement être, ou comprendre, des cellules CD34+ (humaines), en particulier des cellules hématopoïétiques CD34+ (humaines) ou des cellules CD34+ non-hématopoïétiques (humaines). Ces cellules CD34+ (humaines) peuvent par exemple être des cellules du sang périphérique (humain) ou du cordon ombilical (humain) ou d'un tissu humain, en particulier du sang périphérique (humain).

Ces cellules, en particulier ces cellules humaines, à l'exclusion des cellules souches embryonnaires humaines, peuvent avantageusement être, ou comprendre, des cellules souches ou progénitrices, en particulier des cellules multipotentes ou pluripotentes.

Ces cellules ne sont pas, ou ne comprennent pas, de cellules embryonnaires humaines.

Ce milieu de culture cellulaire peut notamment comprendre des composants qui autorisent la multiplication de ces cellules, plus particulièrement qui autorisent leur multiplication tout en les maintenant dans un état non différencié, ou à tout le moins tout en ne leur faisant pas atteindre un état de différentiation terminale.

Ce milieu de culture cellulaire peut comprendre de l'insuline, de la transferrine et du plasma ou sérum (humain).

Ce milieu de culture cellulaire peut comprendre des cellules support (*feeder cells*), telles que des fibroblastes, ou en être dépourvu.

Ce milieu de culture cellulaire peut par exemple être, ou comprendre, le milieu de Dulbecco modifié par Iscove (milieu *Iscove's Modified Dulbecco's Medium* ou IMDM ; voir, par exemple, Iscove, N.N. and Melchers, F. (1978) J. Exper. Med., 147:923), optionnellement complémenté en glutamine ou en peptides contenant de la glutamine.

Plus particulièrement, ce milieu de culture cellulaire peut être, ou comprendre, le milieu IMDM, optionnellement complémenté en glutamine ou en peptides contenant de la glutamine, ce milieu étant dépourvu de fibroblastes et comprenant de l'insuline (recombinante) humaine, de la transferrine (humaine) et du plasma ou sérum (humain).

Par exemple, le milieu de culture peut comprendre :
- de 1 à 50 µg/mL, en particulier de 8 à 12 µg/mL, d'insuline,
- de 100 à 2000 µg/mL, en particulier de 300 à 500 µg/mL, de transferrine, et
- de 1 à 30%, en particulier de 4 à 12%, de sérum ou plasma (humain).

Le milieu de culture peut en outre comprendre de l'héparine, par exemple de 1,5 à 3,5 IU/mL d'héparine.

Le milieu de culture peut en outre comprendre de l'érythropoïétine, notamment de l'érythropoïétine (recombinante) humaine.

Le milieu de culture peut en outre comprendre un ou plusieurs facteurs de croissance, notamment un ou plusieurs facteurs de croissance choisis parmi l'hydrocortisone, le SCF (*Stem Cell Factor*), l'interleukine 3 (IL-3), la thrombopoïétine (TOP), la FLT3 (*Fms-like tyrosine kinase 3*), la BMP4 (*Bone Morphogenetic Protein 4*), la VEGF-A 165 (*Vascular endothelial growth factor* A 165), et l'IL-6.

Le milieu de culture peut notamment comprendre
- de l'hydrocortisone, ou
- du SCF, ou
- du SCF et de l'IL-3, ou
- de l'hydrocortisone et du SCF, ou
- de l'hydrocortisone, du SCF et de l'IL-3, ou
- du SCF, de la TOP, de la FLT3, de la BMP4, de la VEGF-A165, de l'IL-3 et de l'IL-6.

Le milieu de culture peut notamment être un milieu de culture tel que décrit dans WO 2011/101468 (ou dans l'une des demandes de brevets US qui sont issues de, ou basées sur, cette demande internationale PCT, telle que notamment la demande US 2013/0078721 A1).

La cassette peut comprendre le milieu de culture et/ou des cellules tels que ci-dessus décrits.

Selon un mode de réalisation, la cassette comprend le milieu de culture dans la poche, plus particulièrement sous forme congelée, mais ne comprend pas de cellules.

Selon un autre mode de réalisation, la cassette comprend le milieu de culture dans la poche, plus particulièrement sous forme non congelée, et comprend en outre des cellules, en particulier des cellules en culture, notamment des cellules CD34+ (humaines) telles que ci-dessus décrites.

L'invention concerne encore un incubateur de culture cellulaire, comprenant une enceinte thermostatée et un dispositif de support et d'agitation d'une cassette de culture cellulaire selon l'une des revendications précédentes et des moyens de positionnement et de blocage de la cassette de culture cellulaire dans une position donnée dans le dispositif de support.

Dans une réalisation, le dispositif de support et d'agitation comprend un plateau de support de la cassette de culture cellulaire qui est monté rotatif autour d'un axe horizontal autour duquel le plateau est destiné à osciller pour l'agitation et l'homogénéisation du contenu de la poche.

Le dispositif de support et d'agitation peut comprendre une bielle dont une extrémité supérieure est reliée par l'intermédiaire d'un oscillateur au plateau et dont l'extrémité inférieure est reliée à une manivelle entraînée en rotation par l'arbre d'un moteur dont l'axe est sensiblement horizontal.

L'incubateur peut comprendre des moyens de contrôle de la position de la cassette de culture cellulaire dans les moyens de support de manière à vérifier que la cassette est convenablement positionnée sur les moyens de support et que celle-ci peut être utilisée.

Le plateau porte des actuateurs électromécaniques d'ouverture/fermeture des vannes dont une sortie porte des seconds moyens d'accouplement destinés à coopérer avec des premiers moyens d'accouplement des vannes de la cassette lorsque la cassette est dans ladite position donnée.

Selon une autre caractéristique de l'invention, l'incubateur comprend une porte de fermeture étanche d'une ouverture de l'enceinte, cette ouverture comprenant un bord périphérique dans lequel est formé un renfoncement dont la forme est adaptée à recevoir la barrette de support des conduits, la barrette formant un organe d'étanchéité entre la porte et le bord périphérique lorsque la porte est en position de fermeture de l'enceinte.

L'intégration d'une barrette de support des conduits permet de réaliser de manière simple et rapide l'opération de positionnement des conduits au niveau du passage de porte. En effet, contrairement à la technique connue qui consiste à intégrer des gorges de logement des conduits directement dans le bord périphérique, et à les disposer une à une dans des gorges, l'invention propose de réaliser un montage de tous les conduits en une seule étape. De plus, l'étanchéité est réalisée sur la barrette et non plus sur les conduits, ce qui permet de simplifier la réalisation de la fermeture étanche puisque cette barrette peut présenter une structure rigide sur laquelle la porte peut venir s'appuyer.

L'invention concerne également un automate de culture cellulaire comprenant au moins un incubateur du type décrit ci-dessus, et un système informatique de commande incluant des moyens de saisie et d'enregistrement de données et destiné à réguler les conditions de culture dans une enceinte dudit au moins un incubateur et à piloter les vannes de la cassette logée dans l'enceinte.

L'invention concerne également un procédé de culture cellulaire au moyen d'un automate décrit ci-dessus, le procédé comprenant les étapes consistant à :
a) placer une cassette de culture cellulaire telle que décrite précédemment à l'intérieur de l'enceinte de l'incubateur dans une position de décongélation sur le dispositif de support et d'agitation ;
b) alimenter la poche en cellules à cultiver ;
c) placer la cassette sur le dispositif de support et d'agitation dans une position de culture cellulaire ;
d) agiter la cassette de culture cellulaire durant une période de temps donnée pour homogénéiser son contenu ;
e) maintenir la cassette de culture cellulaire dans des conditions d'incubation pendant une durée de plusieurs jours ; et
f) récupérer le contenu de la cassette au moyen de l'une des tubulures de la cassette.

Le procédé comprend encore :
- pendant l'étape d), une ou plusieurs étapes de prélèvement d'échantillon du contenu de la poche, qui sont chacune précédées d'une étape de basculement du plateau de support d'une position horizontale de culture jusqu'à une position inclinée dans laquelle la zone de prélèvement de la poche représente le point le plus bas de la poche.

L'invention sera mieux comprise et d'autres détails, avantages et caractéristiques de l'invention apparaîtront à la lecture de la description suivante faite à titre d'exemple non limitatif, en référence aux dessins annexés dans lesquels :
- la figure 1 est une vue schématique en perspective de l'automate de culture cellulaire selon l'invention, cet automate comportant un incubateurs définissant une enceinte thermostatée qui est ouverte ;
- la figure 2 est une vue schématique en perspective et isolée de l'incubateur ;
- les figures 3A et 3B sont des vues schématiques de côté des moyens de support et d'agitation et de la cassette ;
- la figure 4 est une vue schématique en perspective des moyens de support et d'agitation et de la cassette ;
- la figure 5 est une vue schématique du dessus de la cassette reposant sur les moyens de support et d'agitation ;
- les figures 6A à 6C représentent différentes positions d'utilisation de la cassette dans l'enceinte thermostatée de l'automate ;
- la figure 7 une vue schématique en perspective de la cassette selon l'invention, en direction de la demi-coque supérieure ;
- la figure 8 une vue schématique en perspective de la cassette selon l'invention, en direction de la demi-coque inférieure ;
- la figure 9 est une vue schématique à plus grande échelle de la zone délimitée en pointillée sur la figure 8 ;
- la figure 10 est une vue schématique en perspective de la cassette de la figure 7, la demi-coque supérieure étant retirée ;
- la figure 11 est une vue schématique en perspective et isolée de la demi-coque inférieure,
- la figure 12 est une vue schématique en perspective à plus grande échelle de la zone délimitée pointillée sur la figure 11 ;
- les figures 13 et 14 sont des vues schématiques en perspective de la demi-coque supérieure ;
- la figure 15A est une vue schématique en perspective d'une platine de réception des vannes d'ouverture/fermeture des conduits de la cassette ;
- les figures 15B et 15C sont des vues schématiques en perspective et à plus grande échelle d'une partie seulement de la platine de la figure 15A ;
- les figures 16A, 16B et 16C représentent une première variante d'une vanne utilisable avec la platine de la figure 15A ;
- les figures 17A et 17B représentent une seconde variante d'une vanne utilisable avec la platine de la figure 15A ;
- la figure 18 est une vue schématique en perspective de moyens d'entrainement en rotation de la vanne des figures 16A, 16B et 16C ;
- les figures 19A et 19B sont des vues schématiques en perspective d'un organe d'actionnement de la vanne des figures 17A et 17B ;
- la figure 20 est une vue schématique en perspective d'un système de culture cellulaire formé d'une pluralité d'incubateurs et d'une centrifugeuse
- la figure 21 est un organigramme montrant des étapes d'un procédé de culture cellulaire selon l'invention.

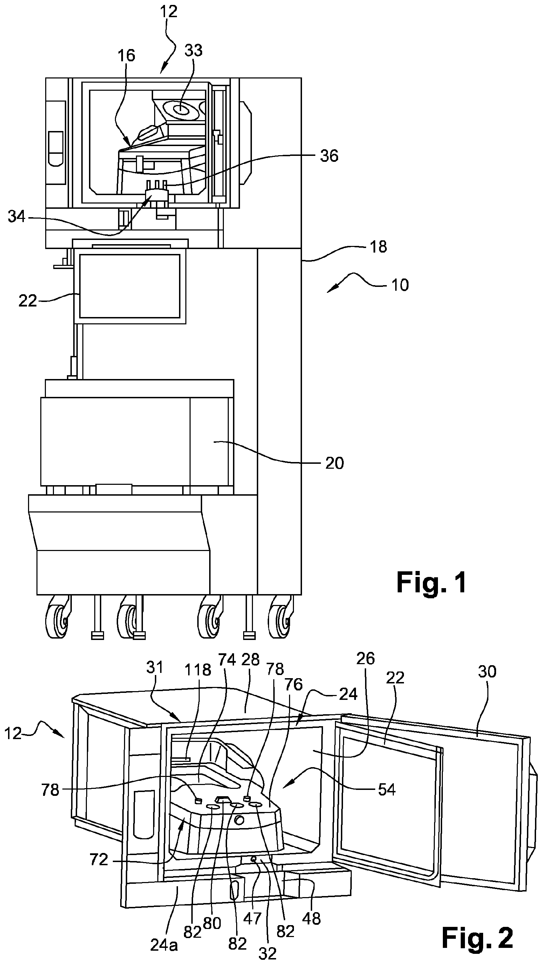

On se réfère tout d'abord aux figures 1 et 2 qui représentent un exemple de réalisation de l'automate 10 de culture cellulaire selon l'invention, cet automate 10 étant par exemple destiné à la culture de cellules souches, à l'exclusion des cellules souches embryonnaires humaines.

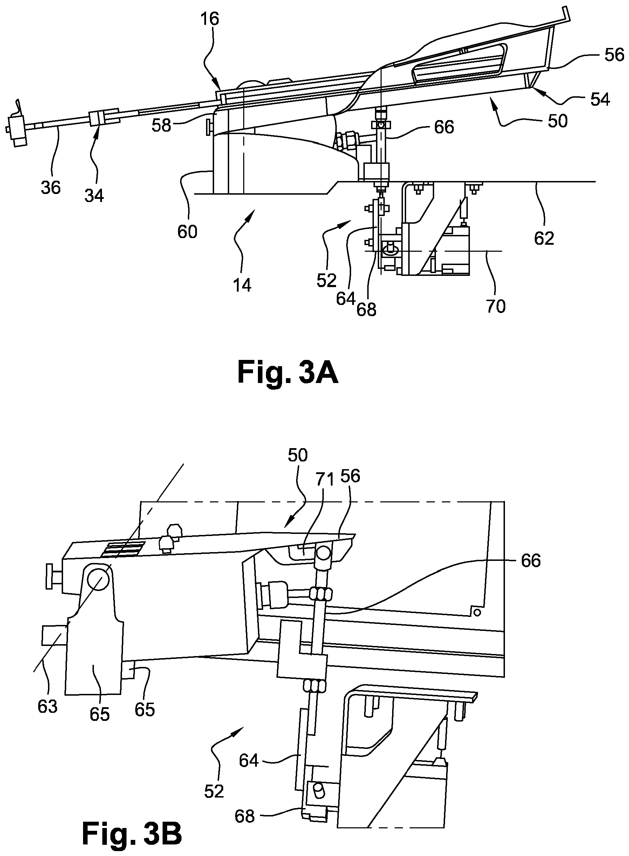

Comme représenté, l'automate 10 comprend essentiellement trois éléments :
- un incubateur 12 à enceinte thermostatée comprenant un dispositif de support et d'agitation 14 d'une cassette de culture cellulaire 16 selon l'invention (figures 1 et 3) ;
- un bâti 18 de support de l'incubateur 12 et d'une centrifugeuse 20, et
- un système informatique relié à l'incubateur 12 et à la centrifugeuse 20 pour la commande de vannes et de pompes et porté par le bâti 18.

De façon typique, le système informatique comprend des moyens de saisie et d'enregistrement de données, des moyens de traitement des données, des moyens d'affichage, et des moyens d'émission de signaux de commande et de pilotage de l'incubateur ainsi que des vannes de la cassette 16 et pompes. De préférence, le système informatique comprend un écran tactile d'affichage 22 et de saisie de données par un opérateur ou utilisateur.

Le contrôle et la traçabilité des étapes du protocole biologique peuvent être assurés par le système informatique et une interface homme-machine (IHM) appropriée, qui permettent notamment :
- d'assurer la traçabilité complète du procédé de fabrication de chaque greffon de cellules souches, des étapes d'intervention des opérateurs sur l'automate, des paramètres de culture comme la température, le taux de C02, et le temps de déroulement de chaque étape et des actions de validation nécessaires à certaines étapes du procédé,
- d'assurer la traçabilité des consommables et réactifs utilisés dans le procédé, via une interface de saisie, des moyens de lecture et de stockage de données de code-barres, et d'étiquettes RFID, et une vérification de cohérence des informations recueillies vis-à-vis d'une base de donnée interne. Cette traçabilité pouvant s'appliquer à la totalité de la chaîne de fabrication, de stockage et de transport des consommables et réactifs, ainsi que sur les conditions matérielles de maintien en température de ces composants.
- d'indiquer de façon systématique à l'utilisateur le détail des étapes du protocole à travers des animations et images illustrant le déroulement des étapes du procédé,
- d'enregistrer de façon sécurisée des données d'identification du patient, les données biologiques liées à la caractérisation des greffons de cellules souches,
- de piloter chacune des fonctions de commande des actionneurs,
- d'enregistrer et interpréter les données des différents capteurs présents sur l'automate ou la cassette fluidique.

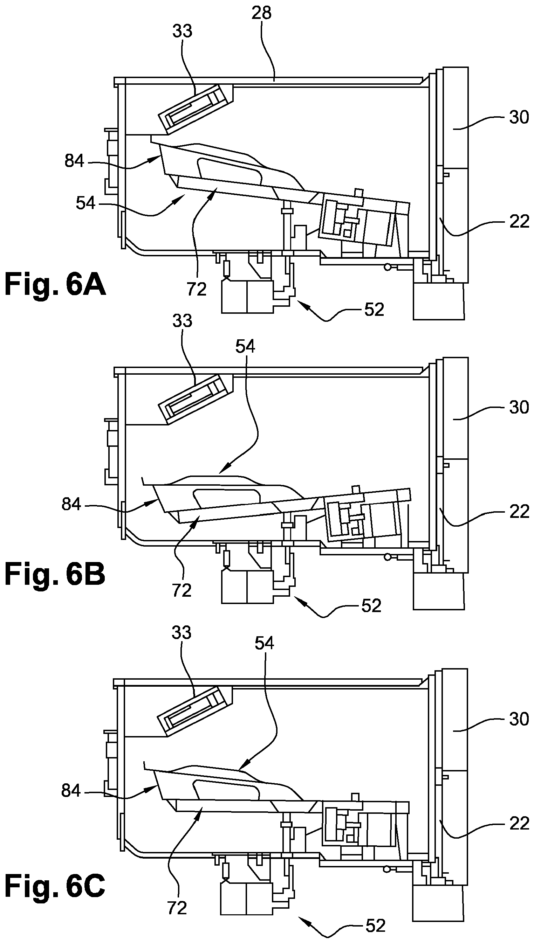

Comme représenté en figure 2, l'incubateur 12 comprend deux portes, une première porte 22 interne destinée à venir s'appliquer sur une première surface 24 de contact entourant l'ouverture 26 de l'enceinte 28 de l'incubateur 12 et une seconde porte 30 externe destinée à venir s'appliquer sur une seconde surface 31 de contact entourant la première surface de contact 24. Les première 22 et seconde 30 portes sont par exemple chacune pivotable autour d'un même axe vertical. L'enceinte 28 loge des moyens de ventilation 33 qui sont agencés en partie supérieure de l'enceinte 28 (figures 1, 6A, 6B et 6C). Ces moyens de ventilation 33 sont dimensionnés et configurés pour permettre une répartition uniforme de la température et des gaz dans l'enceinte de l'incubateur nécessaires à une culture cellulaire efficace et d'avoir une température adaptée (figures 1, 6A, 6B et 6C). Pendant la décongélation du milieu de culture qui est contenu dans la poche, la chaleur dans l'enceinte est ainsi uniformément répartie pour permettre une décongélation rapide du milieu de culture cellulaire.

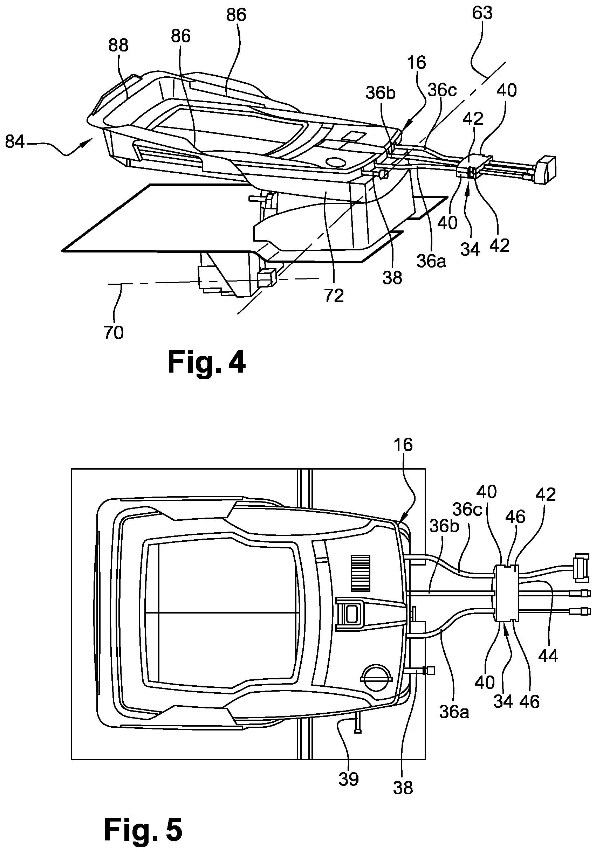

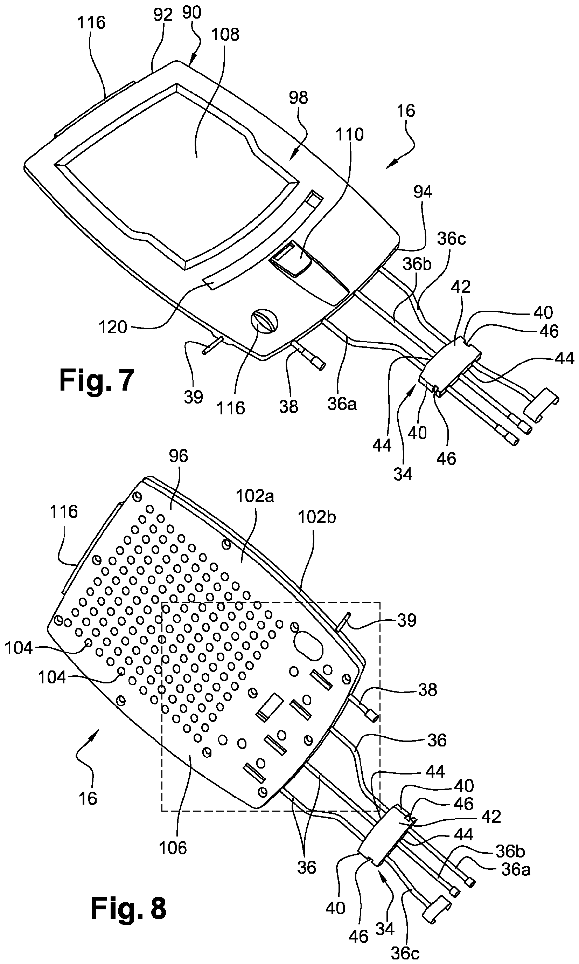

La porte interne 22 peut être réalisée en verre transparent de façon à permettre une observation du contenu de l'enceinte 28 de l'incubateur 12 tout en conservant l'incubateur 12 en position fermée. L'ouverture 26 de l'enceinte 28 comprend un bord périphérique 24, correspondant à la première surface de contact, dont le bord inférieur 24a comporte un renfoncement 32 destiné à recevoir une barrette 34 de maintien des tubulures ou conduits 36, 38 de la cassette 16 qui sera décrite plus en détails ultérieurement, notamment en référence aux figures 7 et 8. La barrette 34, qui est visible aux figures 4, 5, 7 et 8, forme une coque rigide qui peut présenter une forme sensiblement parallélépipédique. Elle peut également comporter deux flancs 40 opposés et reliés l'un à l'autre par des faces 42 inférieure et supérieure. Les conduits ou tubulures 36, 38 s'étendent à étanchéité au travers d'orifices de la barrette 34. Ces orifices sont formés dans des parois 44 sensiblement perpendiculaires aux flancs 40 et aux parois 42 inférieure et supérieure. Chaque flanc 40 de la barrette 34 comprend une rainure 46 parallèle aux faces 42 inférieure et supérieure. Les rainures 46 des flancs 40 sont disposées de manière dissymétrique l'une par rapport à l'autre par rapport à un plan médian de la barrette 34 et parallèle aux deux flancs 40. Ces rainures 46 sont destinées à recevoir des nervures (non visibles) des flancs du renfoncement 32 du bord périphérique inférieure 24a de l'incubateur 12 de manière à assurer un positionnement de la barrette 34 dans le renfoncement 32. Le positionnement dissymétrique des rainures 46 permet de réaliser un détrompage au montage de la barrette 34 dans le renfoncement 32 de l'incubateur 12. Ainsi, de manière plus générale, la barrette peut comprendre des moyens de détrompage au montage de celle-ci dans un renfoncement d'un bord périphérique d'une ouverture de l'incubateur. L'ouverture peut alors être obturée par un organe mobile, telle qu'une porte ou une trappe, qui est déplaçable entre une position d'ouverture de l'incubateur et une position de fermeture de l'ouverture de l'incubateur. La barrette ou coque rigide 34 a de préférence une forme complémentaire à celle du renfoncement 32. Cette forme complémentaire peut être telleque lorsque la barrette 34 est en position dans le renfoncement 32, sa face inférieure 42 vient en contact avec la face de fond du renfoncement 32 et la face supérieure 42 affleure le reste du bord périphérique 24 de l'ouverture 26 de l'enceinte 28 de l'incubateur 12 afin de former une surface sensiblement plane sur laquelle la première porte 22 peut venir en appui à étanchéité. De cette manière, l'étanchéité de la porte interne 22 sur le bord périphérique 24 de l'ouverture 26 de l'enceinte 28 de l'incubateur 12 peut être réalisée de manière simple. De plus, le passage des tubulures 36, 38 ou conduits au travers de la première porte 22 peut être réalisé de manière simple sans qu'il soit nécessaire de manipuler les conduits 36, 38 un à un. Enfin, lorsque la première porte 22 est appliquée sur la barrette 34, l'effort de fermeture s'exerce sur la structure rigide de la barrette 34 et non pas sur les tubulures 36, 38, ce qui évite tout écrasement des tubulures 36, 38 et permet de garantir leur section de passage de fluide. De préférence, le renfoncement 32 peut comprendre un capteur de présence 47 de la barrette 34 permettant de confirmer le bon positionnement de la barrette dans le renfoncement 32 de manière à autoriser la fermeture de la première porte 22 puis de la seconde porte 30.

Comme cela est visible sur la figure 2, la façade de l'enceinte de l'incubateur 12 comprend un autre renfoncement 48 débouchant dans le renfoncement 32 du bord périphérique 24 de l'ouverture 26 de l'enceinte 28. Ce renfoncement 48 est destiné à permettre le passage des conduits 36, 38 ou tubulures au-delà du renfoncement 32 de la première porte 22 et de la seconde porte 30 jusqu'à la centrifugeuse. En pratique, ce second renfoncement 48 peut être obturé par une trappe (non représentée), par exemple coulissante.

Comme représenté en figure 3A, l'enceinte thermostatée 28 comprend un dispositif de support et d'agitation 14 d'une cassette 16 de culture cellulaire selon l'invention. Ce dispositif peut se décomposer en un dispositif de support 50 et un dispositif d'agitation 52 reliés l'un à l'autre. Le dispositif de support 50 comprend un plateau 54 de support ou berceau de support de la cassette 16 et une embase 60. Le plateau 54 comporte une première extrémité 56 agencée au fond de l'enceinte 28 thermostatée et une seconde extrémité 58 opposée à la première extrémité qui est relié à rotation autour d'un axe horizontal 63 à l'embase 60 s'étendant sensiblement verticalement et qui est portée par la paroi 62 formant le plancher de l'enceinte thermostatée 28 (figures 3A et 4). Plus précisément et comme cela est représenté en figure 3B, la première extrémité 56 du plateau 54 est portée et articulée à rotation autour de l'axe 63 sur deux bras 65 s'étendant verticalement depuis le plancher 62 de l'enceinte 28 et formé de part et d'autre du plateau 54.

Le dispositif d'agitation 52 comprend un système du type bielle/manivelle comportant une bielle 64 dont une extrémité supérieure est reliée à une extrémité inférieure d'un oscillateur ou tige verticale oscillante 66 supportant à son extrémité supérieure opposée le plateau 54 par sa face inférieure. L'extrémité inférieure de la bielle 64 est reliée à une manivelle 68 entrainée en rotation par un arbre d'un moteur dont l'axe 70 de rotation est sensiblement horizontal. Cet axe 70 est perpendiculaire à l'axe 63. L'extrémité supérieure 66 de l'oscillateur est reliée au plateau de manière à permettre un déplacement de l'oscillateur dans un plan perpendiculaire à l'axe de rotation 70. Egalement, l'extrémité supérieure de l'oscillateur 66 est reliée à déplacement relativement au plateau support 54 dans la direction de l'axe 70, cette liaison pouvant être réalisée par le montage à coulissement d'un doigt, de l'extrémité supérieur de l'oscillateur 66, dans une fente 71 ou rainure du plateau 54.

Comme représenté sur les figures 3, 4 et 5, l'oscillateur 64 traverse la paroi de plancher 62 de l'enceinte 28 de l'incubateur 12. De préférence, des moyens d'étanchéité, tels qu'un soufflet, sont prévus autour de l'oscillateur 66, au niveau de la zone de traversée de la paroi de plancher 62 de manière à éviter les transferts de chaleur et d'humidité en dehors de l'enceinte 28 thermostatée qui pourraient endommager les éléments mécanique du dispositif d'agitation 52.

Les figures 6A, 6B et 6C représentent trois positions prises par le plateau 54 au cours de son déplacement par les moyens d'agitation. En figure 6A est représentée la position haute du plateau 54 et la figure 6B représentant la position basse du plateau 54. Dans l'une et l'autre de ces deux positions le plateau est incliné par rapport à un plan horizontal. La figure 6C représente une position médiane dans laquelle le plateau 54 est sensiblement horizontal. En fonctionnement, le plateau 54 de support de la cassette est ainsi apte à effectuer une angulation entre ses positions extrêmes (figures 6A et 6C) autour de l'axe 63 (figure 4).

Comme représenté en figure 2, le plateau 54 de support de la cassette 16 est formé d'un cadre 72 comportant une ouverture 74 ou partie creuse et une partie pleine 76. La partie creuse 74, agencée au voisinage de la première extrémité 56 du plateau 54, est destinée à venir en vis-à-vis de la demi-coque inférieure de la cassette qui sera décrite ci-après. La partie pleine 76 est agencée au voisinage de la seconde extrémité 58 du plateau 54. Cette partie pleine 76 comprend des moyens de positionnement et de blocage de la cassette de culture cellulaire dans une position donnée sur le plateau 54 correspondant à une position dans laquelle la cassette 16 repose sur le cadre 72 et permet de réaliser une phase d'incubation. Les moyens de positionnement comprennent deux pions 78 en saillie vers le haut destinés à coopérer avec deux trous borgnes de la cassette 16 (décrit ci-après). Les moyens de blocage de la cassette 16 comprennent un crochet de clipsage 80 ou d'encliquetage avec un crochet de la cassette qui sera décrit ci-après de manière à bloquer la cassette 16 sur le plateau 54. Le crochet 80 de la partie pleine 46 du cadre 72 du plateau 54 est agencé entre les deux pions 78.

Comme cela est visible sur la figure 2, le plateau comprend trois actuateurs électromécaniques 82 d'ouverture/fermeture de vannes, chaque actuateur 82 comprenant une sortie comportant des seconds moyens d'accouplement destinés à coopérer avec des premiers moyens d'accouplement des vannes de la cassette lorsque la cassette 16 repose sur le cadre 72 du plateau 54.

Le cadre 72 est surmonté d'une structure 84 de support de la cassette 16 à distance du cadre 72. Cette structure 84 de support permet de supporter la casette 16 dans une seconde position correspondant à une phase de décongélation. Cette structure support 84 comprend deux ailettes 86 latérales reliées l'une à l'autre par une paroi plane 88 et forment ensemble un plan commun de support de la cassette 16 dans la seconde position. On remarque que les plans de support de la cassette 16 dans la première position et dans la seconde position sont inclinés l'un par rapport à l'autre. La seconde position permet de supporter la cassette 16, emballée hermétiquement sous vide dans une enveloppe, à distance du cadre 72 afin d'éviter toute déchirure de l'enveloppe, généralement réalisée en matière plastique, par les pions 78 lors de la phase de décongélation.

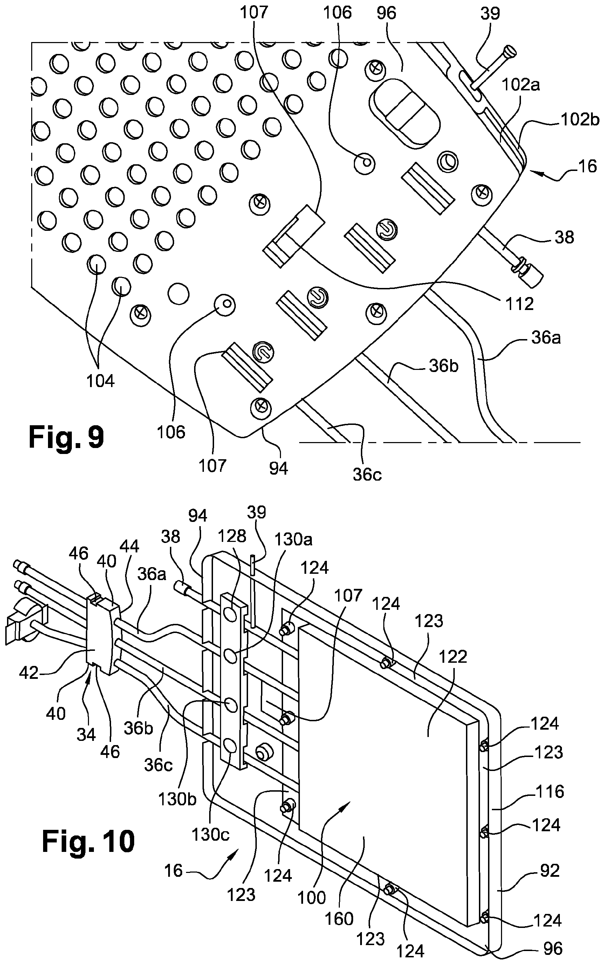

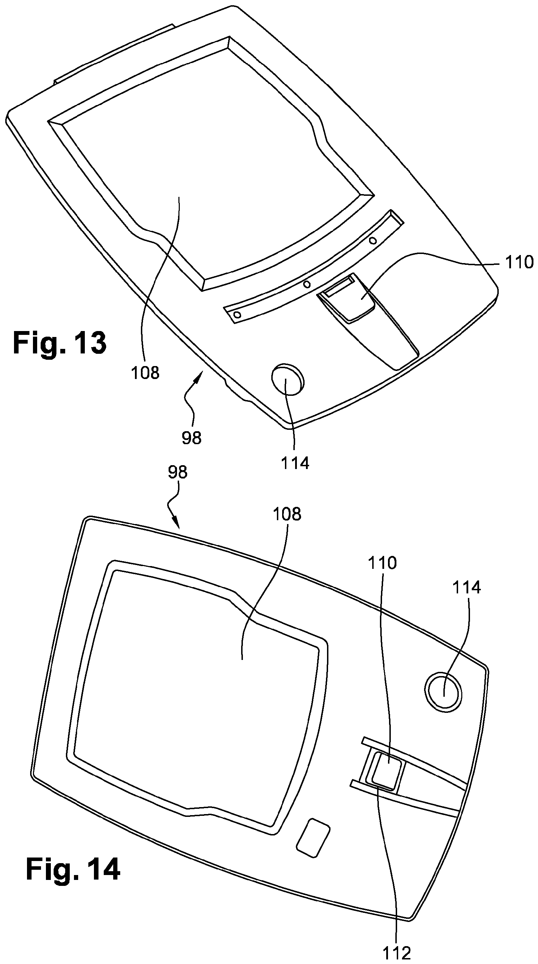

On se réfère maintenant aux figures 7 et 8 qui représentent une cassette 16 de culture cellulaire selon l'invention comprenant un boîtier 90 présentant une forme correspondant sensiblement à celle d'un parallélépipède rectangle comportant une première extrémité 92 et une seconde extrémité 94 en référence au positionnement de la cassette 16 sur le plateau 54. Le boîtier 90 comporte une demi-coque inférieure 96 (figures 9 à 12) et une demi-coque supérieure 98 (figures 13 et 14) solidaires l'une de l'autre, la demi-coque inférieure 96 étant rigide et la demi-coque supérieure 98 étant rigide ou partiellement rigide, c'est-à-dire comprenant des parties flexibles. Les deux demi-coques inférieure 96 et supérieure 98 définissent ensemble un logement dans lequel est fixée une poche 100 souple de culture cellulaire définissant un volume intérieur destiné à être remplit d'un milieu de culture cellulaire.

Les demi-coques inférieure 96 et supérieure 98 sont reliées l'une à l'autre par quatre rebords périphériques 102a, 102b. Les deux demi-coques inférieure 96 et supérieure 98 peuvent être assemblées par clipsage, collage ou soudure de leurs rebords périphériques 102a, 102b. Dans une réalisation préférée, les deux demi-coques inférieure 96 et supérieure 98 sont toutes les deux rigides.

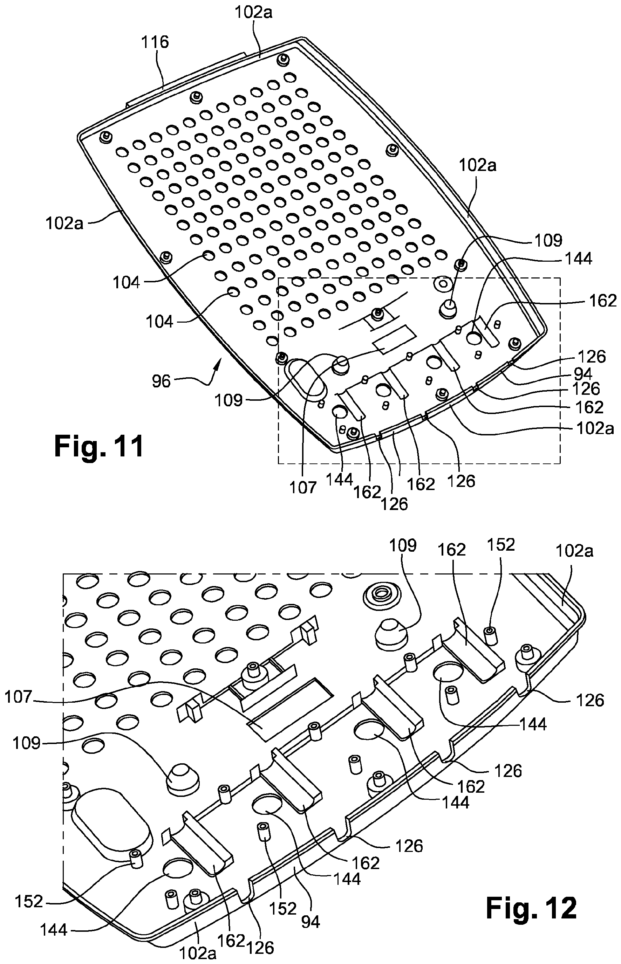

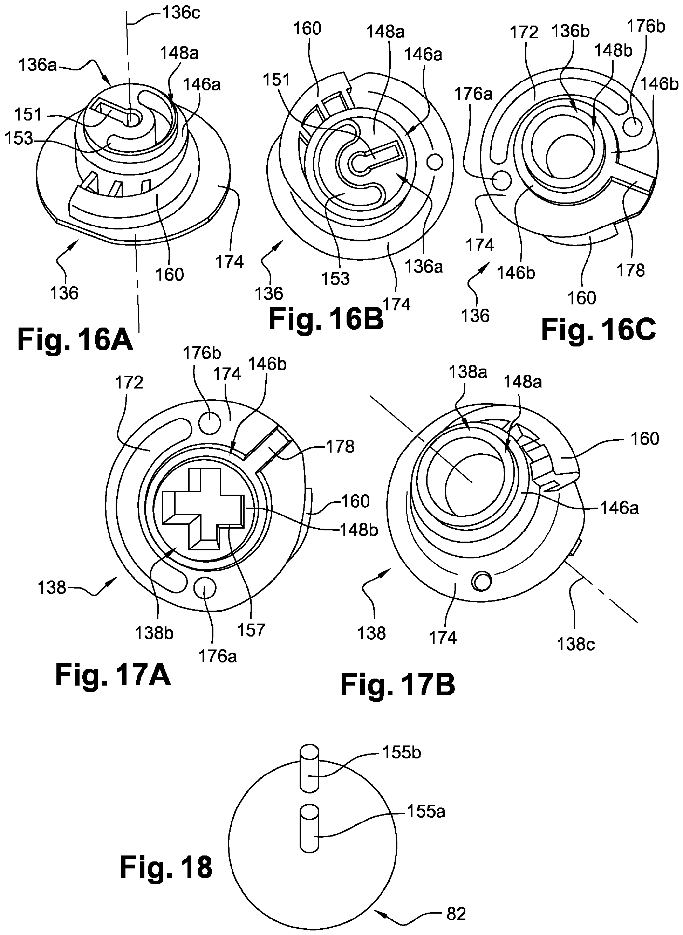

La demi-coque inférieure 96 comprend une pluralité d'orifices 104 ayant de préférence un diamètre inférieur à 20 mm. La cassette 16 a par exemple des dimensions de 405 mm de longueur, 305 mm de largeur et une épaisseur de 25 mm. Elle comprend également deux trous borgnes 106 espacés l'un de l'autre et encadrant une ouverture 107 traversante ou débouchante. Les trous borgnes 106 sont formés par un renfoncement 109 de la demi-coque inférieure 96, s'étendant vers la demi-coque supérieure 98 (figures 11 et 12). La demi-coque supérieure 98 comprend une ouverture ou évidement 108 dont la forme et la dimension sont déterminées pour permettre la propagation d'une ondulation du liquide de la poche 100 dans l'ouverture lorsque la cassette 16 contenant un liquide subit une oscillation autour de l'axe horizontal 63, l'ouverture 108 ou ajour (ou découpe) étant disposée vers le haut (figures 7, 13, 14). Comme cela a été évoqué précédemment, la formation d'un évidement 108 permet de former une ondulation de la surface de la poche 100 située à l'intérieur du boîtier lorsque la cassette 16 est montée dans la seconde position sur le plateau 54 et que celui-ci est déplacé à oscillation comme décrit en référence aux figures 6A, 6B et 6C. La demi-coque supérieure 98 comprend une languette élastique 110 portant un crochet de clipsage 112 avec le crochet 80 du cadre 72 du plateau 54 comme cela a été déjà évoqué précédemment. Le crochet 112 de la demi-coque supérieure 98 est ainsi configuré pour traverser l'ouverture 107 de la demi-coque inférieure 96. Enfin, la demi-coque supérieure 98 comprend également un orifice circulaire 114 d'insertion d'un bouchon d'actionnement 116 manuel d'une vanne dont l'utilisation apparaitra plus clairement en référence aux figures 17 et 18.

On remarque que le rebord périphérique 102a de la demi-coque inférieure 96, situé au niveau de la première extrémité 92 du boîtier 90, comporte une nervure rectiligne 116 qui est destinée à coopérer avec une fente 118 de la structure support 84 du plateau 54 lorsque la cassette 16 de culture cellulaire est montée dans sa première position sur le plateau 54 dans l'enceinte 28 de l'incubateur 12 (figures 8, 9, 11, 12). Dans cette première position, le crochet de clipsage 112 de la demi-coque supérieure 98 traverse l'ouverture 107 de la demi-coque inférieure 96 et coopère avec le crochet 80 du cadre 72 du plateau 54 pour assurer le blocage de la cassette 16 dans cette première position (figures 3, 4 et 5). Pour libérer la cassette 16 et la retirer de l'enceinte 28 d'incubation, il suffit d'appuyer sur la languette 110, ce qui conduit à désengager le crochet 110 de la demi-coque supérieure 98 d'avec le crochet 80 de la du plateau 54.

Une plaquette 120 est fixée par encliquetage dans la demi-coque supérieure et est apte à permettre un maintien des tubulures 36, 38 de la 16 cassette en position repliées sur la face externe de la demi-coque supérieure 98.

La figure 10 représente l'intérieur du boîtier 90, la demi-coque supérieure 98 n'étant pas représentée. Comme cela est visible, la poche 100 de culture de cellulaire présente une forme sensiblement parallélépipédique comportant une première paroi 122 et une seconde paroi (non visible) sensiblement parallèles l'une à l'autre à l'état vide et à l'état remplit de liquide. La première paroi 122 et la seconde paroi sont reliées et rendues solidaires l'une à l'autre par leurs bords périphériques 123, par tout moyen tel que par exemple par soudure ou collage. Dans une réalisation particulière de l'invention, le rapport de la surface de l'une des première paroi 122 ou seconde paroi sur le volume interne de la poche est compris entre 540 et 600 cm²/L, et est de préférence de l'ordre de 580 cm²/L. La distance séparant la première paroi 122 de la seconde paroi est inférieure à 20 mm, de préférence compris entre 10 et 20 mm et encore plus préférentiellement de l'ordre de 14 mm.

La poche 100 de culture cellulaire est fixée sur la demi-coque inférieure 96 par l'intermédiaire de pion 124 en saillie sur la face interne de la demi-coque inférieure 96. Ces pions 124 traversent des orifices des bords 123 périphériques de la poche 100 de culture cellulaire. Le volume intérieur de la poche 100 est en communication avec l'extérieur par l'intermédiaire d'une pluralité de conduits. En particulier, la cassette 16 peut comprendre quatre conduits 36, 38 ou tubulures souples dont trois 36 sont solidaires de la barrette 34 précédemment décrite et un quatrième 38 est indépendant.

Plus particulièrement, la cassette 16 comprend une première 36a et une seconde tubulure 36b dites de prélèvement et une tubulure 36c de vidange. La tubulure 38 correspond à une tubulure d'injection des cellules d'intérêt. Comme cela est visible sur les figures 5, 7, 8, 9 et 10, la cassette comprend également une tubulure 39 de remplissage initial de la poche 100 en milieu de culture. Dans une configuration préférée de la cassette 16 selon l'invention, les quatre tubulures 36a, 36b, 36c, 38 sont équipées de connecteurs pouvant être des connecteurs aseptiques et/ou de soutirage sans aiguille, ces connecteurs pouvant encore comprendre des moyens anti-retour. Dans la suite de la description, lorsque le numéro de référence 36 est utilisé seul, il désignera indifféremment l'une au moins des tubulures 36a, 36b ou 36c.

Comme représenté en figure 10, les tubulures 36, 38 s'étendent au travers d'encoches 126 d'un même rebord périphérique 102a de la demi-coque inférieure 96, à savoir le rebord périphérique 102a formé au niveau de la seconde extrémité 94 du boîtier 90. La tubulure 39 de remplissage s'étend sur l'un des côtés adjacents au côté traversé par les tubulures 36, 38 et est reliée à son extrémité agencée à l'intérieur du boîtier 90 dans une portion de la tubulure 36 agencée fluidiquement entre une vanne 128 et la poche 100.

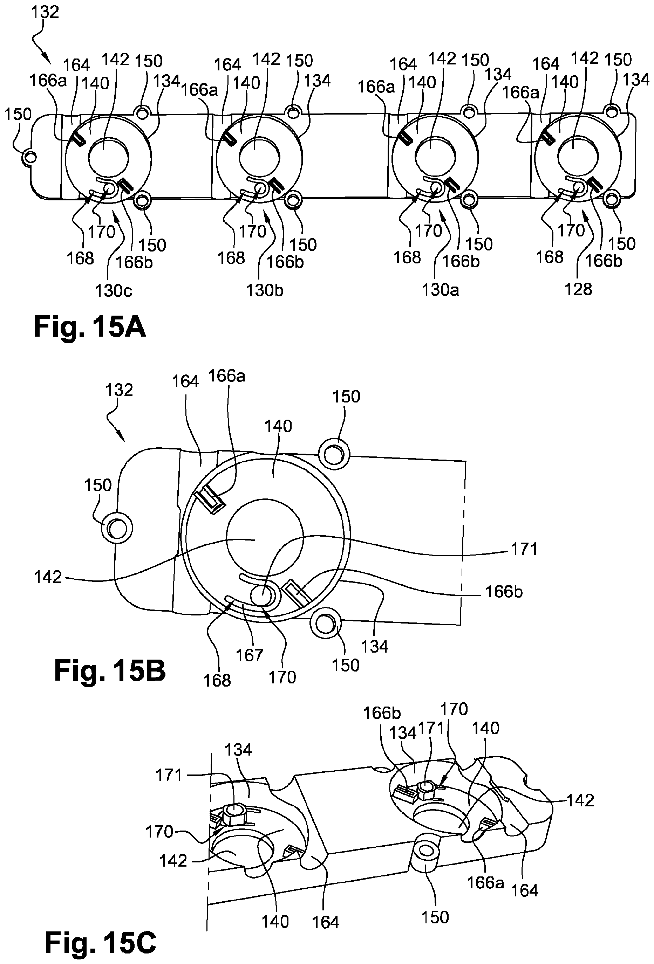

Chacune des tubulures 36, 38 traverse des vannes 128, 130a, 130b, 130c d'ouverture/fermeture de la circulation de fluide au travers des conduits 36, 38, ces vannes à pincement 128, 130 étant agencées dans le boîtier 90. Dans l'exemple représenté, les trois tubulures 36 traversent un premier type de vanne 130, la tubulure 36a traversant la vanne 130a, la tubulure 36b traversant la vanne 130b et la tubulure 36c traversant la vanne 130c, tandis que la quatrième tubulure 38 traverse un second type de vanne 128 (figures 10 et 15A). Plus précisément, le boîtier 90 comprend une platine 132 (figures 15A, 15B et 15C) comportant une pluralité de logement 134 sensiblement cylindrique recevant chacun un organe mobile 136, 138 de pincement d'une tubulure 36, 38 (figures 16 et 17). Cette platine forme ainsi le corps ou partie statorique des vannes 128, 130. Chaque logement 134 de la platine 132 comprend une paroi annulaire de fond 140 délimitant un orifice central 142 aligné avec un orifice 144 de la demi-coque inférieure 96 lorsque la platine 132 est montée dans le boîtier 90 (figures 10 et 12).

Chaque organe mobile 136, 138 comprend une extrémité inférieure 136a, 138a et une extrémité supérieure 136b, 138b en référence aux demi-coques inférieure 96 et supérieure 98 et en référence à l'agencement de la cassette par rapport à la verticale. Bien entendu, il serait possible de désigner de manière plus générale l'extrémité inférieure comme étant une première extrémité et l'extrémité supérieure comme étant une seconde extrémité opposée à la première extrémité. Ainsi, l'extrémité supérieure 136b, 138b de chaque organe mobile 136, 138 comprend un épaulement annulaire 146b destiné à venir en appui selon l'axe de rotation 136c, 138c sur le pourtour d'un orifice 142 d'un logement 134 de la platine 132 et entourant une paroi cylindrique 148b destinée à s'insérer dans l'orifice 142 du logement. Dans le cas présent, la surface 146b de l'organe 136 est en appui sur la surface de fond 140 d'un logement 134 d'une vanne 130a, 130b, 130c. Il est ainsi possible de réaliser un guidage en rotation et un centrage des secondes extrémités 136b, 136a des organes mobiles 136, 138 dans les logements 134 de la platine 132. L'extrémité inférieure 136a, 138a de chaque organe mobile 136, 138 comprend un épaulement annulaire 146a destiné à venir en appui selon l'axe de rotation 136c, 138c sur le pourtour d'un orifice 144 de la demi-coque inférieure 96 et une paroi cylindrique 148a destinée à s'insérer dans l'orifice 144 de la demi-coque inférieure 96. Dans le cas présent, la surface 146b de l'organe 138 est en appui sur la surface de fond 140 d'un logement 134 de la vanne 128. De cette manière, on assure un guidage en rotation et un centrage de chacun des organes mobiles 136, 138 sur la demi-coque inférieure 96 et la platine 142 qui est elle-même solidaire de la demi-coque inférieure 96 par l'intermédiaire d'oreilles 150 traversées par des plots 152 de la face interne de la demi-coque inférieure 96 et grâce à la demi-coque supérieure 98 dont les rebords périphériques 102b sont solidarisés aux rebords périphériques 102a de la demi-coque inférieure 96. Les extrémités inférieures 136a, 138a des organes mobiles 136, 138 débouchent ainsi au travers de la demi-coque inférieure 96 et sont ainsi accessibles depuis la face externe de la demi-coque inférieure 96 comme cela est visible sur la figure 9.

Les organes mobiles 136, tels que représentés en figure 16A, 16B, 16C associés aux tubulures 36, comprennent à leur extrémité inférieure 136a des premiers moyens d'accouplement accessibles depuis l'extérieur du boîtier de manière à coopérer avec les seconds moyens d'accouplement des actuateurs 82 des organes mobiles 136, agencés dans l'incubateur. Les premiers moyens d'accouplement, formés sur la face inférieure de l'organe 136, comprennent ici une fente 151 radiale dont une extrémité radialement interne est traversée par l'axe de rotation 136c et une gorge semi-circulaire 153 centré sur l'axe de rotation 136cLes seconds moyens d'accouplement des actuateurs 82 peuvent par exemple comprendre deux tiges 155a, 155b dont une première 155a est centrée sur l'axe de rotation de l'actuateur 82 et une seconde 155b est décalée radialement par rapport à la première 155a. Lorsque la cassette 16 est montée sur le cadre 72, comme en figure 3A, la première tige 155a s'engage dans l'extrémité radialement interne de la fente 151, la seconde tige 155b s'engageant dans la gorge semi-circulaire 153. On comprend que l'utilisation d'une gorge semi-circulaire 153 permet de garantir une coopération de la seconde tige 153b avec la gorge 153 quelle que soit la position angulaire de l'organe mobile 136 préalablement au montage de la cassette 16 sur le cadre 72. L'intégration d'une fente radiale 151 sur les organes mobiles 136 permet d'actionner manuellement au besoin l'organe mobile 136.

Dans le cas de la vanne 138 des figures 17A et 17B associée à la tubulure 38, les premiers moyens d'accouplement sont formés à l'extrémité supérieure 138b de l'organe mobile 138. Contrairement aux trois organes mobiles 136 des vannes 130, l'organe mobile 138 de la vanne 128 n'est pas actionné par un actuateur électromécanique mais manuellement par un organe ou bouton 154 rotatif comme celui représenté aux figures 18A et 18B. L'accouplement est ici réalisé par un évidement en croix 157 à l'extrémité supérieure 138b de l'organe mobile 138 dans lequel vient s'engager une forme complémentaire 156 en croix du bouton d'actionnement 154. Le bouton 154 est rendu solidaire de la demi-coque supérieure 98 au moyen d'une pluralité de crochets 158 élastiquement déformables venant s'accrocher sur le pourtour interne de l'orifice 114 circulaire de la demi-coque supérieure 98.

Pour réaliser le pincement des tubulures 36, 38, chaque organe mobile 136, 138 comprend une excroissance en spirale 160 autour de l'axe de rotation 136c, 138c de l'organe mobile 136, 138. La surface radialement externe de chaque excroissance 160 forme une surface d'appui sur une tubulure 36, 38. Chaque excroissance 160 présente un plan de symétrie selon un plan perpendiculaire à l'axe de rotation 136c, 138c. Chaque tubulure 36, 38 est logée pour partie dans une gorge rectiligne 162 à section semi-circulaire de la demi-coque inférieure 96 et pour une autre partie dans une gorge rectiligne 164 à section semi-circulaire de la platine 132 de manière à traverser la périphérie d'un logement 134 de la platine 132 dans une direction perpendiculaire à l'axe de rotation 136c, 138c de l'organe mobile (figures 11, 12, 15A, 15B, 15C). Ainsi, au fur et à mesure de la rotation d'un organe mobile 136, 138 d'une vanne 128, 130 l'excroissance 160 assure une ouverture ou une fermeture progressive de la section d'une tubulure 36, 38 selon le sens de rotation de l'organe mobile 136, 138.

Chaque paroi 140 annulaire de fond d'un logement de la platine 132 porte deux organes 166a, 166b de butée en saillie à l'intérieur du logement 134 et qui sont espacés angulairement l'un de l'autre. Une découpe 167 est formée dans chaque paroi 140 annulaire de fond d'un logement 134 de manière à délimiter une lamelle élastique 168 comportant une extrémité libre portant une excroissance en saillie dans le logement 134, cette excroissance comportant une portion convexe 171 destiné à coopérer à glissement avec les organes mobiles 136, 138 (figures 15B, 15C). L'excroissance 170 coopère à glissement avec une gorge 172, ayant une forme en arc de cercle et un fond incurvé concave, de chacun des organes mobiles 136, 138. Pour ce qui concerne l'organe mobile 136 des figures 16A, 16B et 16C, la gorge 172 est formée sur une collerette annulaire radiale 174 agencée axialement entre l'excroissance 160 et l'épaulement annulaire supérieure 146b et sur la face orientée vers l'extrémité supérieure 136b de l'organe mobile 136. De même, pour ce qui concerne l'organe mobile 138 des figures 17A et 17B, la gorge 172 est formée sur une collerette annulaire radiale 174 agencée axialement entre l'excroissance 160 et l'épaulement annulaire supérieure 146b et sur la face orientée vers l'extrémité supérieure 138b de l'organe mobile 138.

Une cavité semi-sphérique 176a, 176b est formée aux extrémités angulaires de chaque gorge 170, 172 de manière à former des positions d'arrêt de l'organe mobile 136, 138 pour l'une correspondant à la position d'ouverture où le fluide peut circuler et pour l'autre correspondant à une position de fermeture où le fluide ne peut pas circuler. On note également que chaque organe mobile 136, 138 comprend un doigt radial 178 prolongeant latéralement l'épaulement supérieur 146b et est en contact avec la face de la collerette 174 portant la gorge 172.. Ainsi, dans la position de blocage de l'écoulement au travers des tubulures 36, 38, l'organe mobile 136, 138 est positionné de sorte que l'excroissance 170 de la lamelle élastique 168 est engagée dans la cavité ou logement 176a de l'organe mobile 136, 138, le doigt radial 178 de l'organe mobile 136, 138 étant agencé en butée en rotation dans le sens anti-horaire, sur la figure 15, contre l'organe 166a de butée. Dans la position de passage de l'écoulement au travers des tubulures 36, 38, l'organe mobile 136, 138 est positionné de sorte que l'excroissance 170 de la lamelle élastique 168 est engagé dans la cavité ou logement 176b de l'organe mobile 136, 138, le doigt radial 178 de l'organe mobile 136, 138 étant agencé en butée en rotation dans le sens horaire, sur la figure 15, contre l'organe 166b de butée. On comprend que la coopération de l'excroissance 170 avec les cavités 176a, 176b permet d'assurer un verrouillage par encliquetage élastique des organes mobiles 136, 138 en position de fermeture ou en position d'ouverture.

On comprend aisément que les vannes décrites précédemment pourraient tout à fait être utilisées dans d'autres dispositifs nécessitant des organes d'ouverture/fermeture de la circulation d'un fluide au travers d'une tubulure. Par conséquent, la présente description vise également tout dispositif intégrant les vannes décrites, tel que par exemple un dispositif comprenant une platine 132 comportant une pluralité de logements 134 dans lesquels des organes rotatifs sont engagés.

Il serait possible d'équiper la cassette 16 et/ou l'incubateur de moyens de vérification automatique du bon positionnement de la cassette 16 dans chacune de ses première et seconde positions. A cette fin, un aimant pourrait être disposé à l'intérieur du boîtier 90, sur la face interne de la demi-coque inférieure 96, cet aimant coopérant avec deux capteurs à effet Hall positionnés sur le plateau de manière adéquate en relation avec les première et seconde positions à détecter.

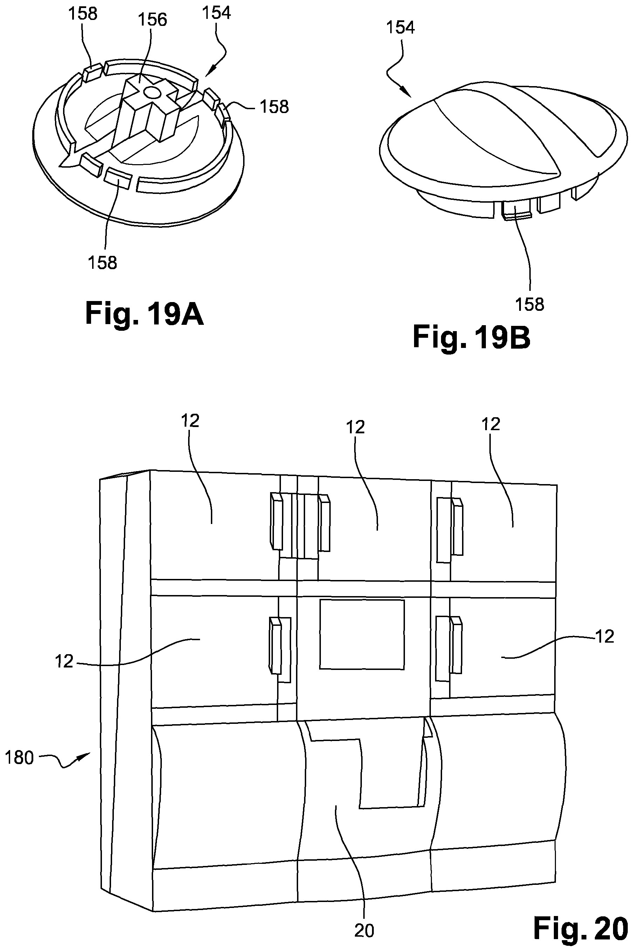

La figure 20 représente un système 180 de culture cellulaire comprenant une pluralité d'incubateurs 12 tels que décrits précédemment et une centrifugeuse 20. Les incubateurs 12 et la centrifugeuse 20 sont commandés par le système informatique.

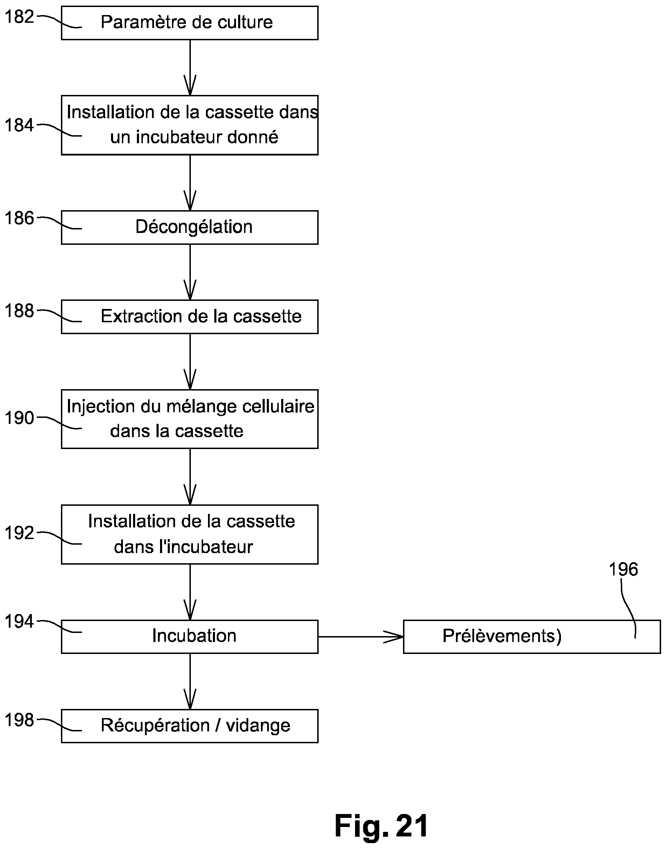

La figure 21 est un organigramme représentant des étapes du procédé de culture cellulaire selon l'invention.

Au préalable du procédé décrit ci-après, la cassette 16 de culture cellulaire est remplit avec un milieu de culture cellulaire en utilisant la tubulure 39 latérale. Cette tubulure 39 est après remplissage immédiatement obturée, par soudure par exemple. La cassette 16 de culture cellulaire est ensuite congelée à plat, c'est-à-dire en position horizontal, pour stockage et utilisation ultérieure.

Une première étape 182 du procédé consiste à saisir et enregistrer au moyen du système informatique des paramètres de culture propres au protocole biologique. La saisie est réalisée par un opérateur, les paramètres saisis étant par exemple l'identification du patient, l'identification de la cassette, etc. Pour faciliter la saisie de ces paramètres, le système informatique peut être équipé d'un lecteur de codes-barres, la cassette pouvant comprendre un code-barres renseignant directement le système informatique avec le numéro et la nature de la cassette. Il est ensuite effectuer une saisie par lecture de code barre ou par saisie manuelle via l'écran tactile des données d'identifications personnelles indiquées sur une étiquette d'un conteneur, telle qu'une poche, des cellules à cultiver.

Dans une seconde étape 184, le système informatique attribue un incubateur donné à l'opérateur après vérification de la disponibilité de l'incubateur en fonction d'un planning de culture cellulaire. L'opérateur installe la cassette 16 de culture cellulaire comprenant un liquide de culture cellulaire dans ledit incubateur désigné, les autres incubateurs étant alors, de préférence, non accessibles, c'est-à-dire les portes 22, 30 des autres incubateurs 12 étant verrouillées.

Dans une troisième étape 186, le procédé consiste à effectuer la décongélation du milieu de culture logé dans la poche souple d'une cassette de culture telle que décrite précédemment. Pour cela, la cassette 16 de culture cellulaire, emballée hermétiquement sous vide dans une enveloppe de protection stérile, est montée dans la seconde position sur le dispositif 52 de support et d'agitation de l'incubateur désigné par le système informatique, c'est-à-dire sur la structure support 84 (figures 3A et 3B). L'opérateur referme les deux portes 22 et 30 et le système informatique initie la procédure de décongélation, les portes 22, 30 étant alors automatiquement verrouillées. Le dispositif de support et d'agitation est actionné de manière à positionner la cassette 16 en position horizontale.

Dans une quatrième étape 188, après décongélation, la cassette 16 de culture cellulaire est extraite de l'incubateur et l'enveloppe externe est retirée dans une zone à activité contrôlée adéquate.

Dans une cinquième étape 190, des cellules d'intérêt, à l'exclusion des cellules souches embryonnaires humaines, sont injectées dans la cassette. Pour cela, la vanne manuelle 128 est positionnée en position ouverte. Il est rappelé que les tubulures 36, 38 sont équipées de moyens anti-retour empêchant le liquide de la poche 100 de s'écouler vers l'extérieur. Les cellules sont injectées dans la poche 100 au moyen de la tubulure 38 puis la vanne 128 est positionnée en position de fermeture et la tubulure 38 obturée avec un bouchon étanche.

Dans une sixième étape 192, la cassette 16 remplit du milieu de culture et des cellules d'intérêt est montée dans sa première position correspondant à la position d'incubation. La barrette 34 est montée dans l'ouverture 32 de la porte 22, les extrémités des tubulures 36 opposées à la poche 100 s'étendant dans l'ouverture 38 et étant reliées au circuit fluidique du système représenté en figure 20.

Le procédé selon l'invention comprend ensuite une étape d'incubation 194 qui peut durer plusieurs jours et par exemple une dizaine de jours. Périodiquement, selon les paramètres du protocole, le contenu de la poche 100 peut être homogénéisé, en déplaçant en oscillation le plateau 54 autour de l'axe 63 comme expliqué précédemment. Cette homogénéisation (durées, fréquence de répétition, amplitude) est déterminée par les paramètres du protocole choisi.

Pendant l'étape d'incubation, l'opérateur peut effectuer un ou plusieurs prélèvements 196 dans la poche 100 (figures 14 et 22). Certains de ces prélèvements peuvent être imposés par le système informatique. Les prélèvements sont réalisés au moyen des tubulures 36a et 36b.

Pour effectuer un premier prélèvement, le système informatique actionne l'organe mobile 136 associé à la vanne associée 130a de manière à autoriser la circulation de fluide au travers de la tubulure 36a. Lorsque le prélèvement est effectué par le système informatique, une opération d'obturation définitive de la tubulure 36a est effectuée de manière à garantir l'asepsie et éviter toute utilisation ultérieure de la tubulure de prélèvement déjà utilisée. Le second prélèvement est effectué de la même façon mais au moyen de la tubulure 36b.

Après chaque sous-étape de prélèvement 196 d'un échantillon dans la poche 100, l'opérateur peut procéder à des analyses dudit échantillon, les résultats de ces analyses pouvant être saisis et enregistrés dans le système informatique par l'opérateur ou bien automatiquement.

Dans une dernière étape du procédé 198, le système informatique procède à une récupération ou vidange du contenu de la poche 100 de la cassette 16.

Enfin, le contenu de la poche subit une étape de purification et de conditionnement dans des seringues stériles en vue d'une utilisation ultérieure.

La demande est également relative à un procédé de culture cellulaire, en particulier de culture de cellules de mammifère, en particulier de cellules humaines, à l'exclusion des cellules souches embryonnaires humaines.

Ces cellules, en particulier ces cellules humaines, à l'exclusion des cellules souches embryonnaires humaines, peuvent avantageusement être, ou comprendre, des cellules CD34+ (humaines), en particulier des cellules hématopoïétiques CD34+ (humaines) ou des cellules CD34+ non-hématopoïétiques (humaines). Ces cellules CD34+ (humaines) peuvent par exemple être des cellules du sang périphérique (humain), ou du cordon ombilical (humain) ou d'un tissu humain, en particulier du sang périphérique (humain).

Ces cellules, en particulier ces cellules humaines, à l'exclusion des cellules souches embryonnaires humaines, peuvent avantageusement être, ou comprendre, des cellules souches ou progénitrices, en particulier des cellules multipotentes ou pluripotentes.

Ces cellules ne sont pas, ou ne comprennent pas, de cellules embryonnaires humaines.

Ces cellules, en particulier ces cellules humaines, à l'exclusion des cellules souches embryonnaires humaines, peuvent avantageusement être, ou comprendre, des cellules souches ou progénitrices humaines CD34+ (multipotentes ou pluripotentes) du sang périphérique mobilisées par un facteur de croissance tel que le G-CSF (*Growth Colony Stimulating Factor*).

Ces cellules, en particulier ces cellules humaines, à l'exclusion des cellules souches embryonnaires humaines, peuvent être ou avoir été prélevées d'un sujet ou patient humain (nouveau-né, enfant, adolescent, adulte ou personne âgée). L'invention est en effet spécialement adaptée aux cellules CD34+ humaines (multipotentes ou pluripotentes) que l'on peut prélever du sang périphérique d'un sujet ou patient humain. Si le patient est destiné à recevoir une chimiothérapie, ces cellules peuvent avoir été collectées avant ou au début de cette chimiothérapie.

Un échantillon biologique contenant les cellules qui sont destinées à être cultivées (par exemple un échantillon de sang (humain), en particulier un échantillon de sang périphérique (humain), de sang de cordon ombilical (humain) ou de tissu humain, en particulier un échantillon de sang périphérique humain) est ou a été prélevé d'un sujet ou patient humain.

Les moyens de la demande présentent l'avantage de ne pas requérir la mise en oeuvre d'une leucaphérèse : un simple échantillon sanguin, par exemple un échantillon de 200 à 250 mL de sang, en particulier de 220 mL de sang, suffit à obtenir un nombre important de cellules, notamment un nombre de cellules qui soit suffisant pour un traitement thérapeutique et/ou préventif, plus particulièrement pour une thérapie cellulaire autologue ou allogénique d'un sujet ou patient (humain), plus particulièrement pour une thérapie cellulaire autologue du sujet ou patient (humain).

C'est notamment le cas pour les cellules CD34+ humaines destinées à un traitement thérapeutique et/ou préventif, plus particulièrement à une thérapie cellulaire autologue ou allogénique du sujet ou patient, plus particulièrement à une thérapie cellulaire autologue du sujet ou patient. Les cellules CD34+ humaines peuvent être des cellules CD34+ humaines non embryonnaires, à l'exclusion des cellules souches embryonnaires humaines. Les cellules CD34+ humaines ne sont pas ou ne comprennent pas de cellules CD34+ embryonnaires.

Les moyens de culture cellulaire de la demande permettent d'obtenir, à partir d'un petit volume de sang (par exemple un échantillon de 200 à 250 mL de sang, en particulier de 220 mL de sang), une population d'au moins 10⁷, ou d'au moins 10⁸, cellules comprenant au moins 70% de cellules CD34+ humaines (non embryonnaires). Par comparaison, pour obtenir une telle quantité de cellules CD34+ humaines directement (sans culture cellulaire) à partir du sang d'un seul sujet ou patient, il serait nécessaire de traiter un volume de 7 à 10 L de sang par leucaphérèse.

Les cellules qui sont destinées à être cultivées, en particulier les cellules CD34+ humaines (non embryonnaires), peuvent être purifiées ou isolées de l'échantillon biologique collecté, notamment d'un échantillon de sang, en particulier d'un échantillon de sang périphérique (par exemple un échantillon de 200 à 250 mL de sang périphérique, en particulier de 220 mL de sang périphérique).

Les cellules peuvent être purifiées de sorte à atteindre un taux minimal en un certain type cellulaire. Par exemple, elles peuvent être purifiées jusqu'à comprendre au moins 70% ou au moins 80% ou au moins 85% ou au moins 90% en cellules CD34+ humaines (non embryonnaires ), en particulier en cellules hématopoïétiques humaines CD34+ (non embryonnaires).

Cet isolement ou purification peut être fait à l'aide de tout moyen connu de la personne du métier.

Par exemple, pour les cellules CD34+ humaines, à l'exclusion des cellules souches embryonnaires humaines, cet isolement ou purification peut être fait à l'aide d'un anticorps monoclonal anti-CD34 humain, par exemple l'anticorps monoclonal murin anti-CD34 humain commercialisé sous le nom de clone QBEnd-10 (code M7165) par DAKO DANEMARK AS (Produktionsvej 42 ; DK-2600 Glostrup ; Danemark). Des systèmes d'isolement ou purification de cellules CD34+ (humaines) sont par ailleurs disponibles dans le commerce, par exemple, le séparateur cellulaire magnétique ISOLEX 300i commercialisé par BAXTER (Deerfield, IL, U.S.A.), ou le kit de microbilles CD34 commercialisé par MILTENYI BIOTECH (2303 Lindbergh Street, Auburn, CA 95602, U.S.A.).

Les cellules purifiées ou isolées sont placées sur ou dans un milieu de culture dont la composition est adaptée à la multiplication de ces cellules. Des exemples de tels milieux de culture, notamment de milieux adaptés à la culture de cellules CD34+ humaines, ont été décrits ci-dessus. Il peut par exemple s'agir d'un milieu de culture tel que ci-dessus décrit, notamment un milieu comprenant de l'insuline humaine, de la transferrine humaine et du plasma ou sérum humain. Ce milieu de culture peut être contenu dans la poche de la cassette de la demande.

Les cellules sont alors placées en incubation, par exemple en plaçant la poche, ou la cassette contenant la poche, dans un incubateur, notamment dans un incubateur de la demande. Le temps d'incubation peut par exemple être de plusieurs jours, notamment de 8 à 10 jours, en particulier de 9 jours, notamment lorsqu'il s'agit d'une culture de cellules CD34+ humaines, à l'exclusion des cellules souches embryonnaires humaines.

Des cellules ou populations de cellules peuvent être obtenues par culture conforme à la demande, telle que décrit ci-dessus.

Ces cellules peuvent notamment être, ou comprendre, des cellules CD34+ (humaines), à l'exclusion des cellules souches embryonnaires humaines, en particulier des cellules CD34+ hématopoïétiques (humaines) ou des cellules CD34+ non-hématopoïétiques (humaines).

Ces cellules CD34+ (humaines) peuvent par exemple être des cellules du sang périphérique (humain), ou du cordon ombilical (humain) ou d'un tissu humain, en particulier du sang périphérique (humain).

Ces cellules peuvent avantageusement être, ou comprendre, des cellules souches ou progénitrices, en particulier des cellules multipotentes ou pluripotentes, à l'exclusion des cellules souches embryonnaires humaines.

Ces cellules ne sont pas, ou ne comprennent pas, de cellules embryonnaires humaines.

Ces cellules peuvent notamment être, ou comprendre, des cellules souches ou progénitrices humaines CD34+ (multipotentes ou pluripotentes) du sang périphérique mobilisées par un facteur de croissance tel que le G-CSF (*Growth Colony Stimulating Factor*).

Ces cellules, notamment les cellules CD34+ (humaines) de la demande, à l'exclusion des cellules souches embryonnaires humaines, peuvent être sous forme pure ou isolée, ou bien être comprise dans une population cellulaire.

Une population cellulaire peut ainsi être une population de cellules humaines non embryonnaires, qui comprend des cellules CD34+ humaines de la demande.

Plus particulièrement, les cellules peuvent notamment être (une population de) des cellules humaines non embryonnaires, qui sont caractérisées en ce que :
- elles sont au nombre d'au moins 10⁷ cellules humaines, plus particulièrement au nombre d'au moins 10⁸, ou d'au moins 1,5.10⁸, ou de 10⁷ à 1,7.10⁸ cellules humaines, et
- elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires).

Ces cellules humaines peuvent en outre présenter toutes le même typage HLA, plus particulièrement le même typage HLA-A, HLA-B, HLA-C et HLA-DR.

Par exemple, les cellules peuvent notamment être (une population de) des cellules humaines hématopoïétiques non embryonnaires, qui sont caractérisées en ce que :
- elles sont au nombre d'au moins 10⁷ cellules hématopoïétiques humaines, plus particulièrement au nombre d'au moins 10⁸, ou d'au moins 1,5.10⁸, ou de 10⁷ à 1,7.10⁸ cellules hématopoïétiques humaines, et
- elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires).

Ces cellules hématopoïétiques humaines peuvent en outre présenter toutes le même typage HLA, plus particulièrement le même typage HLA-A, HLA-B, HLA-C et HLA-DR.

Ces cellules peuvent présenter une ou plusieurs caractéristiques, qui les distinguent de cellules naïves.

Le terme « naïve » signifie dans ce contexte qu'il s'agit de cellules qui ont été directement obtenues d'un sujet ou patient humain, et qui ont éventuellement pu être purifiées, mais qui n'ont pas été cultivées.

En effet, il a été observé qu'après culture conforme à la demande (par exemple sur un milieu de culture et/ou dans une poche ou cassette tels que décrits ci-dessus), la population de cellules humaines obtenue diffère de la population naïve initialement placée en culture, par un ou plusieurs des aspects suivants :
- un plus grand diamètre moyen (diamètres de 11,2 ± 0,5 µm, en particulier de 11,18 ± 0,49 µm),
- une plus forte proportion de cellules CD34- CD14+ (monocytes, lesquels peuvent avoir un impact positif sur l'efficacité d'une greffe cellulaire),
- une proportion plus forte (mais qui n'atteint pas 100%) de cellules CD34+ CD33- (CD33 étant le marqueur de la lignée myéloïde),
- une proportion plus faible (mais restant néanmoins supérieure à 10%) de cellules CD34+ CD133+ (marqueur des progéniteurs endothéliaux).

Plus particulièrement, les cellules de la demande peuvent être des cellules humaines, à l'exclusion des cellules souches embryonnaires humaines, qui présentent une ou plusieurs des caractéristiques suivantes :
1/ elles sont au nombre d'au moins 10⁷ cellules humaines, plus particulièrement au nombre d'au moins 10⁸, ou d'au moins 1,5.10⁸, ou de 10⁷ à 1,7.10⁸ cellules humaines,
2/ elles présentent toutes le même typage HLA, plus particulièrement le même typage HLA-A, HLA-B, HLA-C et HLA-DR,
3/ elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
4/ elles présentent un diamètre cellulaire 11,2 ± 0,5 µm, en particulier de 11,18 ± 0,49 µm,
5/ elles comprennent des cellules CD34- CD14+ (humaines) dans une proportion de 1 à 12%, en particulier de 2 à 10% ou de 3,8 à 10%,
6/ elles comprennent des cellules CD34+ CD33- (humaines) dans une proportion de plus de 10% ou de plus de 20% ou de plus de 25% (et avantageusement de moins de 100%, en particulier de moins de 60% ou de moins de 50% ou de moins de 40% ou de moins de 35%),
7/ elles comprennent des cellules CD34+ CD133+ (humaines) dans une proportion de moins de 60% ou de moins de 50% (et avantageusement de plus de 20%, en particulier de plus de 25% ou de plus de 30%).

Selon un mode de réalisation, les cellules sont des cellules humaines, à l'exclusion des cellules souches embryonnaires humaines, qui présentent au moins deux de ces sept caractéristiques, en particulier au moins trois, ou au moins quatre, ou au moins cinq, ou au moins six de ces sept caractéristiques, ou bien présentent les sept caractéristiques.

Par exemple, les cellules peuvent être des cellules hématopoïétiques humaines, qui présentent une ou plusieurs des caractéristiques suivantes :
1/ elles sont au nombre d'au moins 10⁷ cellules hématopoïétiques humaines, plus particulièrement au nombre d'au moins 10⁸, ou d'au moins 1,5.10⁸, ou de 10⁷ à 1,7.10⁸ cellules hématopoïétiques humaines,
2/ elles présentent toutes le même typage HLA, plus particulièrement le même typage HLA-A, HLA-B, HLA-C et HLA-DR,
3/ elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
4/ elles présentent un diamètre cellulaire 11,2 ± 0,5 µm, en particulier de 11,18 ± 0,49 µm,
5/ elles comprennent des cellules CD34- CD14+ (humaines) dans une proportion de 1 à 12%, en particulier de 2 à 10% ou de 3,8 à 10%,
6/ elles comprennent des cellules CD34+ CD33- (humaines) dans une proportion de plus de 10% ou de plus de 20% ou de plus de 25% (et avantageusement de moins de 100%, en particulier de moins de 60% ou de moins de 50% ou de moins de 40% ou de moins de 35%),
7/ elles comprennent des cellules CD34+ CD133+ (humaines) dans une proportion de moins de 60% ou de moins de 50% (et avantageusement de plus de 20%, en particulier de plus de 25% ou de plus de 30%).

Selon un mode de réalisation, les cellules sont des cellules hématopoïétiques humaines, qui présentent au moins deux de ces sept caractéristiques, en particulier au moins trois, ou au moins quatre, ou au moins cinq, ou au moins six de ces sept caractéristiques, ou bien présentent les sept caractéristiques.

Notamment, les cellules peuvent être des cellules humaines, à l'exclusion des cellules souches embryonnaires humaines, qui présentent une ou plusieurs des caractéristiques suivantes :
1/ elles sont au nombre d'au moins 10⁷ cellules humaines, plus particulièrement au nombre d'au moins 10⁸, ou d'au moins 1,5.10⁸, ou de 10⁷ à 1,7.10⁸ cellules humaines,
2/ elles présentent toutes le même typage HLA, plus particulièrement le même typage HLA-A, HLA-B, HLA-C et HLA-DR,
3/ elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
4/ elles présentent un diamètre cellulaire 11,2 ± 0,5 µm, en particulier de 11,18 ± 0,49 µm,
5/ elles comprennent des cellules CD34- CD14+ (humaines) dans une proportion de 1 à 12%, en particulier de 2 à 10% ou de 3,8 à 10%.

Selon un mode de réalisation, les cellules sont des cellules humaines, à l'exclusion des cellules souches embryonnaires humaines, qui présentent au moins deux de ces cinq caractéristiques, en particulier au moins trois, ou au moins quatre de ces cinq caractéristiques, ou bien présentent les cinq caractéristiques.

Par exemple, les cellules peuvent être des cellules hématopoïétiques humaines, qui présentent une ou plusieurs des caractéristiques suivantes :
1/ elles sont au nombre d'au moins 10⁷ cellules hématopoïétiques humaines, plus particulièrement au nombre d'au moins 10⁸, ou d'au moins 1,5.10⁸, ou de 10⁷ à 1,7.10⁸ cellules hématopoïétiques humaines,
2/ elles présentent toutes le même typage HLA, plus particulièrement le même typage HLA-A, HLA-B, HLA-C et HLA-DR,
3/ elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
4/ elles présentent un diamètre cellulaire 11,2 ± 0,5 µm, en particulier de 11,18 ± 0,49 µm,
5/ elles comprennent des cellules CD34- CD14+ (humaines) dans une proportion de 1 à 12%, en particulier de 2 à 10% ou de 3,8 à 10%.

Selon un mode de réalisation, les cellules sont des cellules hématopoïétiques humaines, qui présentent au moins deux de ces cinq caractéristiques, en particulier au moins trois, ou au moins quatre de ces cinq caractéristiques, ou bien présentent les cinq caractéristiques.

Plus particulièrement, les cellules peuvent être des cellules humaines, à l'exclusion des cellules souches embryonnaires humaines, qui présentent les caractéristiques suivantes :
- elles sont au nombre d'au moins 10⁷ cellules humaines, plus particulièrement au nombre d'au moins 10⁸, ou d'au moins 1,5.10⁸, ou de 10⁷ à 1,7.10⁸ cellules humaines, et
- elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
et qui présentent en outre au moins l'une des (ou les deux) caractéristiques suivantes :
- elles présentent un diamètre cellulaire 11,2 ± 0,5 µm, en particulier de 11,18 ± 0,49 µm,
- elles comprennent des cellules CD34- CD14+ (humaines) dans une proportion de 1 à 12%, en particulier de 2 à 10% ou de 3,8 à 10%.

Par exemple, les cellules de la demande peuvent être des cellules hématopoïétiques humaines, qui présentent les caractéristiques suivantes :
- elles sont au nombre d'au moins 10⁷ cellules hématopoïétiques humaines, plus particulièrement au nombre d'au moins 10⁸, ou d'au moins 1,5.10⁸, ou de 10⁷ à 1,7.10⁸ cellules hématopoïétiques humaines, et
- elles comprennent au moins 70% de cellules CD34+ humaines (multipotentes ou pluripotentes, et non embryonnaires), plus particulièrement au moins 80%, ou au moins 85% ou au moins 90% de cellules CD34+ humaines (multipotentes ou pluripotentes, et non embryonnaires),
et qui présentent en outre au moins l'une des (ou les deux) caractéristiques suivantes :
- elles présentent un diamètre cellulaire 11,2 ± 0,5 µm, en particulier de 11,18 ± 0,49 µm,
- elles comprennent des cellules CD34- CD14+ (humaines) dans une proportion de 1 à 12%, en particulier de 2 à 10% ou de 3,8 à 10%.

Les cellules de cette population peuvent toutes présenter le même typage HLA-A, HLA-B, HLA-C et HLA-DR.

Les cellules de cette population peuvent en outre comprendre :
- des cellules CD34+ CD33- (humaines) dans une proportion de plus de 10% ou de plus de 20% ou de plus de 25% (et avantageusement de moins de 100%, en particulier de moins de 60% ou de moins de 50% ou de moins de 40% ou de moins de 35%),
- des cellules CD34+ CD133+ (humaines) dans une proportion de moins de 60% ou de moins de 50% (et avantageusement de plus de 20%, en particulier de plus de 25% ou de plus de 30% de cellules CD34+ CD133+).

Alternativement ou complémentairement, les cellules peut présenter une ou plusieurs caractéristiques identiques (ou non significativement différentes) de celles de cellules naïves, notamment de cellules humaines naïves, notamment de cellules hématopoïétiques humaines naïves.

En effet, il a été observé qu'après culture conforme à la demande (par exemple sur un milieu de culture et/ou dans une poche cassette tels que décrits ci-dessus), la population de cellules humaines obtenue (notamment la population de cellules hématopoïétiques humaines obtenue) peut présenter des caractéristiques communes à celles de la population naïve de cellules (hématopoïétiques) initialement placée en culture.

Plus particulièrement, les cellules peuvent être des cellules (notamment des cellules hématopoïétiques), qui, par rapport à des cellules naïves (notamment des cellules hématopoïétiques naïves), plus particulièrement par rapport aux cellules (naïves) initialement placées en culture, présentent une ou plusieurs des caractéristiques suivantes :
1/ un nombre ou proportion identique (ou non significativement différent) d'épitopes CD34,
2/ une capacité identique (ou non significativement différente) à se diviser, en particulier une longueur relative de télomère identique (ou non significativement différente),
3/ une même absence d'anomalie chromosomique et de polyploïdie (ou bien les mêmes anomalies chromosomiques ou polyploïdies, dans les mêmes proportions),
4/ une viabilité identique (ou non significativement différente), en particulier une viabilité des cellules CD34+ identique (ou non significativement différente), par exemple une viabilité d'au moins 90% ou d'au moins 95% des cellules CD34+,
5/ une proportion identique (ou non significativement différente) de cellules CD34- CD2+/CD3+,
6/ une proportion identique (ou non significativement différente) de cellules CD34- CD19+/CD20+,
7/ une proportion identique (ou non significativement différente) de cellules CD34- CD56+,
8/ une proportion identique (ou non significativement différente) de cellules CD34- CD15+.

Selon un mode de réalisation, les cellules de la demande présentent au moins deux, ou au moins trois, ou au moins quatre, ou au moins cinq, ou au moins six, ou au moins sept de ces huit caractéristiques, ou bien présentent ces huit caractéristiques.

Plus particulièrement, les cellules de la demande peuvent être des cellules (notamment des cellules hématopoïétiques), qui, par rapport à des cellules (hématopoïétiques) naïves, plus particulièrement par rapport aux cellules (naïves) initialement placées en culture, présentent une ou plusieurs des caractéristiques suivantes :
1/ un nombre ou proportion identique (ou non significativement différent) d'épitopes CD34,
2/ une capacité identique (ou non significativement différente) à se diviser, en particulier une longueur relative de télomère identique (ou non significativement différente),
3/ une même absence d'anomalie chromosomique et de polyploïdie (ou bien les mêmes anomalies chromosomiques ou polyploïdies, dans les mêmes proportions).

Selon un mode de réalisation, les cellules présentent au moins deux de ces trois caractéristiques, ou bien présentent les trois caractéristiques.

Les moyens de culture cellulaire de la demande correspondent essentiellement à des moyens d'amplification cellulaire *ex vivo.* Ils présentent l'avantage d'apporter un nombre important (plus particulièrement un nombre thérapeutiquement suffisant) de cellules autologues ou allogéniques, en particulier de cellules autologues (notamment de cellules CD34+ autologues ou allogéniques, en particulier de cellules CD34+ autologues) à partir d'un petit échantillon de sang périphérique d'un patient ou sujet, sans introduire de modifications délétères sur ces cellules.

Le Tableau 1 ci-dessous donne une illustration des caractéristiques d'une population de cellules obtenues du sang périphérique d'une cohorte de volontaires sains humains avant et après mise en oeuvre d'une culture dans une poche de culture conforme à la demande (culture de 9 jours sur le milieu IMDM complémenté en glutamine et dépourvu de fibroblastes, et contenant 0,01mg/mL d'insuline, 330 µg/ml de transferrine, et 5% (V/V) de sérum humain, de cellules isolées par sédimentation et purifiées par immuno-affinité sur les billes magnétiques anti-CD34):

**Tableau 1 :**

| Cohorte de 71 volontaires sains humains mâles âgés de 21 à 57 ans | Population de cellules CD34+ | |
|---|---|---|
| | Population naïve (telle qu'obtenue sans culture des cellules) | Exemple de population obtenue après culture conforme à la demande (valeur moyenne par patient, après culture des cellules) |
| Echantillon biologique = 220 mL de sang périphérique par patient | | |
| Nombre de cellules CD34+ * | De 5,1.10⁶ à 40,9.10⁶ | De 1.10⁷ à 1,7.10⁸ |
| Viabilité des cellules CD34+ | Supérieure à 95% | Supérieure à 95% |
| Diamètre cellulaire des CD34+ | 8,8 ± 0,1 µm | 11,18 ± 0,49 µm |
| Pourcentage de cellules CD34+ (après purification) | Au moins 70% (ou au moins 80%, ou au moins 85%, ou au moins 90%) | Au moins 70% (ou au moins 80%, ou au moins 85%, ou au moins 90%) |
| Cellules CD34-CD14+ | De 0,2 à 0,8% | De 3,8 à 10% |
| Cellules CD34+ | 4,2% | 33% |
| CD33- | | |
| Cellules CD34+ CD133+ | 75,8% | 44,5% |
| Nombre d'épitopes CD34+ par cellule | 14 804 ± 2 294 | 15 363 ± 2 730 |
| Longueur relative des télomères | 9,17 ± 1,32 % | 8,35 ± 2,07 % |
| Anomalie chromosomique | aucune | aucune |
| Polyploïdie | aucune | aucune |
| Cellules CD34-CD2+/CD3+ | De 0 à 0,3 % | De 0 à 1 % |
| Cellules CD34-CD19+/CD20+ | De 0 à 0,6 % | De 0,1 à 1 % |
| Cellules CD34-CD56+ | 0% | De 0 à 1 % |
| Cellules CD34-CD15+ | De 2 à 5,8 % | De 2,5 à 5 % |

| | | |
|---|---|---|
| * : valeurs issues d'une cohorte de 8 volontaires. | | |

Une fois la culture terminée, les cellules obtenues, plus particulièrement les cellules CD34+ (humaines) obtenues, peuvent être collectées.

Les cellules collectées peuvent être purifiées ou isolées, plus particulièrement purifiées. Notamment, les cellules CD34+ peuvent être purifiées, par exemple à l'aide des moyens de purifications disponibles à la personne du métier tels que ci-dessus indiqués.

Les cellules collectées et éventuellement purifiées peuvent être placées dans une composition, notamment une composition pharmaceutique, en particulier une composition pharmaceutique liquide, par exemple une solution pharmaceutique non-toxique et isotonique pour l'être humain. La composition ou solution qui en résulte est un objet de la demande.

La composition peut comprendre un véhicule physiologiquement acceptable pour l'être humain.

La composition peut être formulée pour être administrable, plus particulièrement injectable, dans un être humain. Elle peut donc être liquide et comprendre des composés non-toxiques pour l'être humain, de préférence des composés non-toxiques à une concentration isotonique pour l'être humain.

Ainsi, en sus des cellules, la composition peut par exemple comprendre une solution tampon non-toxique pour l'être humain, telle qu'une solution de tampon phosphate salin (*Phosphate Buffer Saline* ou PBS), ou une solution tampon de gluconate et/ou acétate.

La composition peut en outre comprendre au moins un additif non toxique pour l'être humain, par exemple une protéine du sérum humain ou du plasma humain, notamment de l'albumine de sérum humain (*Human Serum Albumin* ou HSA).

La composition ou solution peut être conservée jusqu'à utilisation, par exemple par conservation au froid (congélation).

Les moyens de la demande peuvent être utilisés pour un traitement cellulaire (à visée thérapeutique et/ou préventive), notamment pour un traitement cellulaire autologue ou allogénique, avantageusement un traitement cellulaire autologue.

Ce traitement cellulaire peut comprendre l'administration à un patient ou sujet :
- de cellules de la demande, notamment de cellules humaines comprenant des cellules CD34+ (humaines), à l'exclusion des cellules souches embryonnaires humaines, telles que ci-dessus décrites, ou
- d'une composition ou solution pharmaceutique contenant ces cellules.

Ce patient ou sujet peut être le même que celui sur lequel les cellules ont été initialement prélevées pour procéder à la culture cellulaire (traitement ou thérapie cellulaire autologue). Alternativement, ce patient ou sujet peut être différent de celui sur lequel les cellules ont été initialement prélevées pour procéder à la culture cellulaire (traitement ou thérapie cellulaire allogénique).

Le traitement cellulaire, plus particulièrement le traitement cellulaire autologue ou allogénique (en particulier autologue), peut notamment être le traitement ou la prévention d'une insuffisance cardiaque ou d'une pathologie non cardiaque.

L'insuffisance cardiaque peut notamment être :
- une insuffisance cardiaque qui comprend une insuffisance ventriculaire, plus particulièrement une insuffisance ventriculaire gauche,
- plus particulièrement une insuffisance cardiaque avec altération de la fonction systolique,
- plus particulièrement une insuffisance cardiaque avec altération de la fonction contractile d'un ventricule, en particulier du ventricule gauche,
- plus particulièrement une insuffisance cardiaque avec augmentation du volume télésystolique associé à une diminution de la fraction d'éjection du ventricule gauche (*Left Ventricular Ejection Fraction* ou LVEF).

Il peut notamment s'agir d'une insuffisance cardiaque de niveau Il ou supérieur selon la classification de la New York Heart Association (NYHA) [*The* Criteria Committee of the New York Heart Association. 1994. Nomenclature and Criteria for Diagnosis of Diseases of the Heart and Great Vessels. (9ème édition). Boston : Little, Brown & Co., pages 253-256], plus particulièrement d'une insuffisance cardiaque de niveau Il ou supérieur (selon la classification de la NYHA) qui est induite par régurgitation mitrale.

L'insuffisance cardiaque peut notamment être une insuffisance cardiaque induite par
- une hypertension artérielle,
- une atteinte valvulaire,
- une maladie congénitale,
- une angine de poitrine réfractaire (par exemple, une angine de poitrine réfractaire au traitement par ContrePulsion ExtraCorporelle Améliorée (CPECA) ou au traitement de neurostimulation (en particulier à la NeuroStimulation Transcutanée (NST) ou à la Stimulation de la Moelle Épinière (SMÉ), ou au traitement par chélation (en particulier au traitement par administration d'EDTA),
- une cardiomyopathie, en particulier
   - une cardiomyopathie ischémique, en particulier un infarctus du myocarde, plus particulièrement un infarctus aigu du myocarde,
   - une cardiomyopathie dilatée,
   - une cardiomyopathie hypertrophique,
   - une cardiomyopathie restrictive,
   - une Cardiomyopathie Ventriculaire Droite Arythmogène (CVDA).

L'insuffisance cardiaque peut notamment être une insuffisance cardiaque induite par une cardiomyopathie, en particulier par
- une cardiomyopathie ischémique, en particulier un infarctus du myocarde, plus particulièrement un infarctus aigu du myocarde,
- une cardiomyopathie dilatée,
- une cardiomyopathie hypertrophique,
- une cardiomyopathie restrictive,
- une Cardiomyopathie Ventriculaire Droite Arythmogène (CVDA).

L'insuffisance cardiaque peut notamment être une insuffisance cardiaque induite par une cardiomyopathie, en particulier par une cardiomyopathie ischémique, en particulier un infarctus du myocarde, plus particulièrement un infarctus aigu du myocarde.

L'insuffisance cardiaque peut être traitée ou prévenue, notamment en diminuant voire supprimant l'insuffisance cardiaque et/ou en limitant le développement de l'insuffisance cardiaque.

Une insuffisance cardiaque induite par une cardiomyopathie ischémique, en particulier par un infarctus du myocarde, plus particulièrement par un infarctus aigu du myocarde, peut être traitée ou prévenue notamment en diminuant voire supprimant cette insuffisance cardiaque et/ou en limitant le développement de cette insuffisance cardiaque.

En effet, les cellules (notamment les cellules CD34+) obtenues par culture conforme à la demande et éventuellement par purification, ou une composition ou solution pharmaceutique contenant ces cellules, peuvent être utilisées pour un ou plusieurs des buts suivants :
- diminuer voire faire disparaître des dommages myocardiques post-ischémiques, notamment la nécrose post-infarctus et/ou la taille de la cicatrice post-infarctus,
- régénérer partiellement ou complètement la structure et/ou revasculariser le myocarde ou la zone infarcie,
- améliorer voire restaurer la fonction ventriculaire, en particulier la fonction ventriculaire gauche, notamment la contractilité ventriculaire, en particulier la contractilité ventriculaire gauche, par exemple pour diminuer le volume télésystolique en association avec une augmentation de la fraction d'éjection du ventricule gauche (*Left Ventricular Ejection Fraction* ou LVEF) (notamment pour atteindre une LVEF de plus de 45%).

Est plus particulièrement visée le traitement ou la prévention d'une insuffisance cardiaque induite par un infarctus (aigu) du myocarde, plus particulièrement un infarctus (aigu) du myocarde dû à une maladie coronarienne ou à une crise cardiaque. Cette utilisation est plus particulièrement destinée :
- aux patients qui présentent une altération de la fonction ou contractilité ventriculaire gauche, et
- aux patients
   - qui présentent un infarctus aigu du myocarde survenant *de novo* après un premier incident ou syndrome cardiaque (ce premier incident ou syndrome cardiaque ayant éventuellement donné lieu à une implantation de stent(s) et à une angioplastie coronarienne transluminale percutanée (*Percutaneous Transluminal Coronary Angioplasty* ou PTCA), et/ou éventuellement à un pontage aorto-coronarien (*Coronary Artery Bypass Graft Surgery* ou CABG) et
   - qui au moins 10 jours après ce premier incident cardiaque (par exemple, au moins 3 mois ou au moins 6 mois après ce premier incident ou syndrome cardiaque) présentent une fraction d'éjection du ventricule gauche (*Left Ventricular Ejection Fraction* ou LVEF) égale ou inférieure à 45%.

L'administration des cellules, de cellules ou d'une composition ou solution pharmaceutique de la demande peut être par exemple pratiquée quelques semaines, notamment de 3 à 6 semaines après, un accident ischémique.

Le traitement ou la prévention d'une insuffisance cardiaque peut comprendre une cardiomyoplastie cellulaire (par implantation des cellules administrées dans la zone cardiaque infarcie).

Ce traitement ou prévention peut comprendre l'administration d'une population de cellules (notamment d'une population de cellules humaines comprenant des cellules CD34+, à l'exclusion des cellules souches embryonnaires humaines), de cellules (notamment CD34+ humaines, à l'exclusion des cellules souches embryonnaires humaines) de la demande, ou d'une composition ou solution de la demande, en les injectant par chirurgie ou par cathéter.

L'injection par chirurgie peut être une injection directement dans la zone infarcie, par exemple à l'occasion d'un pontage aorto-coronarien (*Coronary Artery Bypass Graft Surgery* ou CABG).

L'injection par cathéter peut notamment être une injection par voie percutanée transfémorale.

Le cathéter peut notamment être un cathéter d'injection ou de perfusion intracoronarienne, épicardique ou transendocardique.

Le cathéter d'injection ou de perfusion peut par exemple être muni être muni d'une aiguille sous forme d'hélice tel que le cathéter HELIX^{®} commercialisé par BIOCARDIA^{®} (125 Shoreway Road, Suite B, San Carlos, CA 94070, U.S.A.), qui peut être couplé à un système de guidage à 2 dimensions (2D) tel que un guidage 2D fluoroscopique.

Le cathéter d'injection ou de perfusion peut par exemple être le cathéter MYOSTAR^{®} commercialisé par BIOSENSE WEBSTER, INC. (15715 Arrow Highway; Irwindale ; CA 91706 ; U.S.A.).

Le cathéter d'injection ou de perfusion peut par exemple être couplé à un système de guidage à 3 dimensions (3D) tel que le système de cartographie cardiaque 3D électromagnétique NOGA^{®} XP commercialisé par BIOLOGICS DELIVERY SYSTEMS GROUP (CORDIS Corp., Diamond Bar, CA, U.S.A.).

Une ou plusieurs chimiokines, en particulier une ou plusieurs chimiokines de la famille CXC, en particulier la (ou au moins la) chimiokine CXCL12 (*Stromal cell-Derived Factor-1* ou SDF-1), peuvent être administrées, plus particulièrement injectées au patient ou sujet. Cette administration ou injection peut être simultanée, conjointe ou différée dans le temps par rapport à l'administration, plus particulièrement à l'injection, d'une population de cellules de la demande (notamment d'une population de cellules humaines comprenant des cellules CD34+, à l'exclusion des cellules souches embryonnaires humaines), de cellules (notamment CD34+ humaines, à l'exclusion des cellules souches embryonnaires humaines) de la demande, ou d'une composition ou solution de la demande.

Les utilisations des moyens de la demande ne sont pas limitées au traitement ou à la prévention d'une insuffisance cardiaque induite par une cardiomyopathie ischémique, ou d'une insuffisance cardiaque induite par un traitement chimio-thérapeutique. Les moyens de la demande (notamment les moyens CD34+ de la demande) peuvent alternativement être utilisés pour traiter ou prévenir des pathologies non cardiaques.

Il peut s'agir d'une pathologie non cardiaque, qui n'est pas induite par une intervention cardiaque.

Les moyens de la demande peuvent en effet être utilisés pour traiter une myélosuppression, notamment une myélosuppression induite par le traitement d'un lymphome, plus particulièrement d'un lymphome non hodgkinien, d'un lymphome hodgkinien, d'une leucémie lymphoïde chronique. Les moyens de la demande peuvent alors être utilisés pour implanter chez le patient, notamment dans sa moelle osseuse, par voie intraveineuse des cellules hématopoïétiques (autologues ou provenant d'un autre sujet) de la demande, notamment une population de cellules hématopoïétiques (autologues ou allogéniques) comprenant des cellules CD34+, telle que ci-dessus décrite. Les cellules implantées sont alors destinées à régénérer la moelle osseuse du patient.

Les moyens de la demande peuvent alternativement être utilisés pour traiter ou prévenir une pathologie induisant une dégénérescence du cartilage, telle que l'arthrose. Les moyens de la demande peuvent alors être utilisés pour implanter chez le patient, notamment dans la zone arthrosique, des cellules (autologues ou provenant d'un autre sujet) de la demande, notamment une population de cellules comprenant des cellules CD34+, telle que ci-dessus décrite. Les cellules implantées sont alors destinées à régénérer le cartilage du patient.

Les moyens de la demande peuvent alternativement être utilisés pour traiter ou prévenir une insuffisance hépatique, notamment une insuffisance hépatique chronique, en particulier une insuffisance hépatique chronique non alcoolique (par exemple pour prévenir le développement d'un carcinome hépatocellulaire). Les moyens de la demande peuvent alors être utilisés pour implanter chez le patient, notamment dans la zone hépatique, des cellules (autologues ou provenant d'un autre sujet) de la demande, notamment une population de cellules (autologues) comprenant des cellules CD34+, telle que ci-dessus décrite. Les cellules implantées sont alors destinées à régénérer le foie du patient.

Le terme "comprenant", avec lequel "incluant" ou "contenant" est synonyme, est un terme ouvert, et n'exclut pas la présence d'un ou plusieurs élément(s), ingrédient(s) ou étape(s) de méthode additionnel(s) qui ne serait(seraient) pas explicitement indiqué(s), tandis que le terme "consistant" ou "constitué" est un terme fermé, qui exclut la présence de tout autre élément additionnel, étape, ou ingrédient qui ne serait pas explicitement exposé. Le terme "consistant essentiellement" ou " essentiellement constitué" est un terme partiellement ouvert, qui n'exclut pas la présence d'un ou plusieurs élément(s), ingrédient(s) ou étape(s) additionnel(s), dans la mesure où ce(s) élément(s), ingrédient(s) ou étape(s) additionnel(s) n'affecte(nt) pas matériellement les propriétés de base de l'invention.

Par conséquent, le terme "comprenant" (ou "comprend(comprennent)") inclut les termes "consistant", "constitué", aussi bien que les termes "consistant essentiellement" et " essentiellement constitué".

## Revendications

1. Cassette (16) de culture cellulaire comprenant :
- un boîtier (90) au moins partiellement rigide et définissant intérieurement un espace intérieur dans lequel est agencée et fixée une poche (100) de culture cellulaire définissant un volume intérieur,
- des conduits (36, 38) reliés chacun à une extrémité au volume intérieur de la poche (100) et ayant chacun une seconde extrémité située à l'extérieur du boîtier (90),
- des vannes (128, 130) d'ouverture/fermeture de la circulation de fluide au travers des conduits (36, 38) qui sont montées sur le boîtier (90).

2. Cassette selon la revendication 1, dans laquelle les conduits (36, 38) sont formés par des tubulures souples et au moins certaines des vannes (128, 130) comprennent un organe mobile (136, 138) de pincement de la tubulure sur un élément de structure (132) du boîtier (90).

3. Cassette selon la revendication 2, dans laquelle chaque organe mobile (136, 138) comprend un axe (136c, 138c) de rotation et la périphérie radialement externe de chaque organe mobile (136, 138) comprend au moins une surface (160) en spirale autour de l'axe (136c, 138c) de rotation formant une surface de pincement de la tubulure (36, 38) de manière à faire varier une section de passage de fluide au travers d'une tubulure (36, 38) au fur et à mesure de la rotation de l'organe (136, 138).

4. Cassette selon la revendication 3, dans laquelle les organes mobiles (136, 138) sont montés dans des logements (134) d'une platine (132) agencée à l'intérieur du boîtier (90) et solidaire du boîtier (90), chaque logement (134) recevant un organe mobile.

5. Cassette selon l'une des revendications 2 à 4, dans laquelle chaque organe mobile (136, 138) comprend une extrémité (136a, 138b) accessible depuis l'extérieur du boîtier (90) et pourvue de premiers moyens d'accouplement (154) en rotation destinés à coopérer avec des seconds moyens d'accouplement en rotation d'un actuateur.

6. Cassette selon la revendication 4 ou les revendications 4 et 5, **caractérisée en ce que** les organes mobiles sont centrés et guidés à rotation à une extrémité dans un orifice du boîtier et à une extrémité opposée dans un orifice de la platine.

7. Cassette selon l'une des revendications 2 à 6, dans laquelle la poche (100) présente une forme sensiblement parallélépipédique comportant une première paroi souple (122) et une seconde paroi souple sensiblement parallèles l'une à l'autre à l'état vide et à l'état remplit de liquide.

8. Cassette selon l'une des revendications 1 à 7, dans laquelle le boîtier (90) présente une forme correspondant sensiblement à celle d'un parallélépipède rectangle et comporte une demi-coque inférieure (96) et une demi-coque supérieure (98) solidaires l'une de l'autre, la demie-coque inférieure (96) étant rigide et la demi-coque supérieure (98) étant rigide ou flexible.

9. Cassette selon la revendication 8, dans laquelle la demi-coque supérieure (98) comprend une ouverture (108) ou évidement central dont la forme et la dimension sont déterminées pour permettre la propagation d'une ondulation du liquide de la poche (100) dans l'ouverture (108) lorsque la cassette (16) contenant un liquide subit une oscillation autour d'un axe horizontal, l'ouverture étant disposée vers le haut.

10. Cassette selon la revendication 8 ou 9, dans laquelle les conduits (36, 38) s'étendent au travers de l'un des rebords périphériques (102a, 102b) de l'une des demi-coques inférieure (96) ou supérieure (98).

11. Cassette selon l'une des revendications 1 à 10, comprenant une barrette (34) de support des conduits (36, 38), agencée à l'extérieur du boîtier (90) et traversée par au moins certains des conduits (36, 38).

12. Incubateur de culture cellulaire, comprenant une enceinte (28) thermostatée et un dispositif de support (14) et d'agitation d'une cassette de culture cellulaire selon l'une des revendications précédentes et des moyens de positionnement et de blocage de la cassette (16) de culture cellulaire dans une position donnée dans le dispositif de support, le dispositif (14) de support et d'agitation comprenant un plateau (54) de support de la cassette (16) de culture cellulaire, ledit plateau (54) portant des actuateurs électromécaniques (82) d'ouverture/fermeture des vannes (128, 130) dont une sortie porte des seconds moyens d'accouplement destinés à coopérer avec des premiers moyens d'accouplement (154) des vannes (128, 130) de la cassette lorsque la cassette (16) est dans ladite position donnée.

13. Incubateur selon la revendication 12, dans lequel le plateau (54) est monté rotatif autour d'un axe horizontal (63) autour duquel le plateau (54) est destiné à osciller pour l'agitation et l'homogénéisation du contenu de la poche (100).

14. Incubateur selon la revendication 13, dans lequel le dispositif (14) de support et d'agitation comprend une bielle (64) dont une extrémité supérieure est reliée par l'intermédiaire d'un oscillateur au plateau (54) et dont l'extrémité inférieure est reliée à une manivelle (68) entraînée en rotation par l'arbre d'un moteur dont l'axe (70) est sensiblement horizontal.

15. Incubateur selon l'une des revendications 12 à 14, comprenant une porte (22) de fermeture étanche d'une ouverture (26) de l'enceinte (28), cette ouverture (26) comprenant un bord périphérique (24) dans lequel est formé un renfoncement (32) dont la forme est adaptée à recevoir la barrette (34) de support des conduits d'une cassette selon la revendication 15, la barrette (34) formant un organe d'étanchéité entre la porte (22) et le bord périphérique lorsque la porte est en position de fermeture de l'enceinte (28).

16. Automate de culture cellulaire comprenant au moins un incubateur (12) selon l'une des revendications 12 à 14, et un système informatique de commande incluant des moyens de saisie et d'enregistrement de données et destiné à réguler les conditions de culture dans une enceinte dudit au moins un incubateur et à piloter les vannes de la cassette logée dans l'enceinte.

17. Procédé de culture cellulaire au moyen d'un automate selon la revendication 16, **caractérisé en ce qu'**il comprend les étapes suivantes :
a) placer une cassette (16) de culture cellulaire selon l'une des revendications 1 à 11 à l'intérieur de l'enceinte de l'incubateur (12) dans une position de décongélation sur le dispositif de support et d'agitation (14);
b) alimenter la poche (100) en cellules à cultiver ;
c) placer la cassette (16) sur le dispositif de support et d'agitation (14) dans une position de culture cellulaire ;
d) agiter la cassette (16) durant une période de temps donnée pour homogénéiser son contenu ;
e) maintenir la cassette (16) dans des conditions d'incubation pendant une durée de plusieurs jours ; et
f) récupérer le contenu de la cassette (16) au moyen de l'une des tubulures de la cassette (16).

18. Procédé selon la revendication 17, **caractérisé en ce qu'**il comprend :
- pendant l'étape d), une ou plusieurs étapes de prélèvement (196) d'échantillon du contenu de la poche, qui sont chacune précédées d'une étape de basculement du plateau (54) de support d'une position horizontale de culture jusqu'à une position inclinée dans laquelle la zone de prélèvement de la poche (100) représente le point le plus bas de la poche (100).

## Patentansprüche

1. Zellkulturkassette (16), enthaltend:
- ein Gehäuse (90), das zumindest teilweise starr ausgebildet ist und innen einen Innenraum definiert, in dem ein ein Innenvolumen definierender Zellkulturbeutel (100) angeordnet und befestigt ist,
- Leitungen (36, 38), die jeweils an einem Ende mit dem Innenvolumen des Beutels (100) verbunden sind und jeweils ein zweites Ende haben, das sich außerhalb des Gehäuses (90) befindet,
- Ventile (128, 130) zum Öffnen/Schließen der Fluidzirkulation durch die Leitungen (36, 38), die an dem Gehäuse (90) angebracht sind.

2. Kassette nach Anspruch 1,
wobei die Leitungen (36, 38) aus nachgiebigen Schläuchen gebildet sind und zumindest einige der Ventile (128, 130) ein bewegliches Organ (136, 138) zum Anklemmen des Schlauchs an ein Strukturelement (132) des Gehäuses (90) aufweisen.

3. Kassette nach Anspruch 2,
wobei jedes bewegliche Organ (136, 138) eine Drehachse (136c, 138c) aufweist und der radial äußere Umfang jedes beweglichen Organs (136, 138) zumindest eine Fläche (160) aufweist, die spiralförmig um die Drehachse (136c, 138c) verläuft und eine Klemmfläche für den Schlauch (36, 38) bildet, um einen Fluiddurchtrittsquerschnitt durch einen Schlauch (36, 38) je nach Drehung des Organs (136, 138) zu verändern.

4. Kassette nach Anspruch 3,
wobei die beweglichen Organe (136, 138) in Aufnahmen (134) einer Platine (132) gelagert sind, die im Inneren des Gehäuses (90) angeordnet und fest mit dem Gehäuse (90) verbunden ist, wobei jede Aufnahme (134) ein bewegliches Organ aufnimmt.

5. Kassette nach einem der Ansprüche 2 bis 4,
wobei jedes bewegliche Organ (136, 138) ein Ende (136a, 138b) aufweist, das von außerhalb des Gehäuses (90) zugänglich ist und mit ersten Drehkopplungsmitteln (154) versehen ist, die dazu bestimmt sind, mit zweiten Drehkopplungsmitteln eines Aktuators zusammenzuwirken.

6. Kassette nach Anspruch 4 oder nach den Ansprüchen 4 und 5,
**dadurch gekennzeichnet, dass** die beweglichen Organe an einem Ende in einer Öffnung des Gehäuses und an einem entgegengesetzten Ende in einer Öffnung der Platine zentriert und drehbar geführt sind.

7. Kassette nach einem der Ansprüche 2 bis 6,
wobei der Beutel (100) eine im Wesentlichen quaderförmige Gestalt mit einer ersten nachgiebigen Wand (122) und einer zweiten nachgiebigen Wand aufweist, die in leerem Zustand und in mit Flüssigkeit gefülltem Zustand im Wesentlichen parallel zueinander verlaufen.

8. Kassette nach einem der Ansprüche 1 bis 7,
wobei das Gehäuse (90) eine Form aufweist, die im Wesentlichen der eines rechteckigen Quaders entspricht, und eine untere Halbschale (96) und eine obere Halbschale (98) umfasst, die fest miteinander verbunden sind, wobei die untere Halbschale (96) starr und die obere Halbschale (98) starr oder flexibel ausgeführt ist.

9. Kassette nach Anspruch 8,
wobei die obere Halbschale (98) eine zentrale Öffnung (108) oder Aussparung aufweist, deren Form und Abmessung so bestimmt sind, dass sich eine Wellenbewegung der Flüssigkeit aus dem Beutel (100) in die Öffnung (108) ausbreiten kann, wenn die eine Flüssigkeit enthaltende Kassette (16) eine Schwingung um eine horizontale Achse erfährt, wobei die Öffnung nach oben gerichtet ist.

10. Kassette nach Anspruch 8 oder 9,
wobei sich die Leitungen (36, 38) durch einen der Umfangsränder (102a, 102b) einer aus unterer Halbschale (96) oder oberer Halbschale (98) hindurch erstrecken.

11. Kassette nach einem der Ansprüche 1 bis 10,
enthaltend einen Steg (34) zum Abstützen der Leitungen (36, 38), der außerhalb des Gehäuses (90) angeordnet ist und von zumindest einigen der Leitungen (36, 38) durchquert wird.

12. Inkubator für Zellkulturen, mit einer thermostabilisierten Kammer (28) und einer Vorrichtung (14) zum Halten und Schütteln einer Zellkulturkassette nach einem der vorhergehenden Ansprüche und Mitteln zum Positionieren und Sichern der Zellkulturkassette (16) in einer gegebenen Position in der Haltevorrichtung, wobei die Halte- und Schüttelvorrichtung (14) eine Platte (54) zum Halten der Zellkulturkassette (16) aufweist, wobei die Platte (54) elektromechanische Aktuatoren (82) zum Öffnen/Schließen der Ventile (128, 130) trägt, von denen ein Auslass zweite Kopplungsmittel trägt, die dazu bestimmt sind, mit ersten Kopplungsmitteln (154) der Ventile (128, 130) der Kassette zusammenzuwirken, wenn sich die Kassette (16) in der gegebenen Position befindet.

13. Inkubator nach Anspruch 12,
wobei die Platte (54) um eine horizontale Achse (63) drehbar gelagert ist, um die die Platte (54) zum Schütteln und Homogenisieren des Inhalts des Beutels (100) schwingen soll.

14. Inkubator nach Anspruch 13,
wobei die Halte- und Schüttelvorrichtung (14) eine Koppelstange (64) umfasst, deren oberes Ende über einen Oszillator mit der Platte (54) verbunden ist und deren unteres Ende mit einer Kurbel (68) verbunden ist, die von einer Motorwelle mit im Wesentlichen horizontaler Achse (70) drehend mitgenommen wird.

15. Inkubator nach einem der Ansprüche 12 bis 14,
enthaltend eine Tür (22) zum abgedichteten Verschließen einer Öffnung (26) der Kammer (28), wobei diese Öffnung (26) einen Umfangsrand (24) aufweist, in dem eine Vertiefung (32) ausgebildet ist, deren Form dazu geeignet ist, den Steg (34) zum Abstützen der Leitungen einer Kassette nach Anspruch 15 aufzunehmen, wobei der Steg (34) ein Dichtungsorgan zwischen Tür (22) und Umfangsrand bildet, wenn die Tür in Schließposition der Kammer (28) ist.

16. Zellkulturautomat mit zumindest einem Inkubator (12) nach einem der Ansprüche 12 bis 14 und einem Steuercomputersystem, das Mittel zur Erfassung und Aufzeichnung von Daten enthält und dazu bestimmt ist, die Kulturbedingungen in einer Kammer des zumindest einen Inkubators zu regeln und die Ventile der in der Kammer aufgenommenen Kassette anzusteuern.

17. Verfahren zur Zellkultivierung mittels eines Automaten nach Anspruch 16,
**dadurch gekennzeichnet, dass** es die folgenden Schritte umfasst:
a) Anordnen einer Zellkulturkassette (16) nach einem der Ansprüche 1 bis 11 innerhalb der Kammer des Inkubators (12) in einer Auftauposition an der Halte- und Schüttelvorrichtung (14);
b) Beschicken des Beutels (100) mit zu kultivierenden Zellen;
c) Anordnen der Kassette (16) an der Halte- und Schüttelvorrichtung (14) in einer Zellkulturposition;
d) Schütteln der Kassette (16) für eine gegebene Zeitdauer, um deren Inhalt zu homogenisieren;
e) Halten der Kassette (16) unter Inkubationsbedingungen für eine Dauer von mehreren Tagen; und
f) Gewinnen des Inhalts der Kassette (16) mithilfe eines der Schläuche der Kassette (16).

18. Verfahren nach Anspruch 17,
**dadurch gekennzeichnet, dass** es umfasst:
während Schritt d) einen oder mehrere Schritte der Entnahme (196) von Proben aus dem Inhalt des Beutels, denen jeweils ein Schritt des Kippens der Halteplatte (54) aus einer horizontalen Kulturposition in eine geneigte Position vorausgeht, in welcher der Entnahmebereich des Beutels (100) die tiefste Stelle des Beutels (100) darstellt.

## Claims

1. Cell culture cassette (16) comprising:
- a housing (90) at least partially rigid and internally defining an interior space in which is arranged and fixed a cell culture bag (100) defining an interior volume,
- conduits (36, 38) each connected at one end to the inner volume of the bag (100) and each having a second end located outside the housing (90),
- valves (128, 130) for opening/closing the fluid flow through the conduits (36, 38), that are mounted on the housing (90) .

2. A cassette according to claim 1, wherein the conduits (36, 38) are formed by flexible tubing and at least some of the valves (128, 130) include a movable member (136, 138) for pinching the tubing against a structural member (132) of the housing (90).

3. A cassette according to claim 2, wherein each movable member (136, 138) comprises a rotation axis (136c, 138c) and the radially outer periphery of each movable member (136, 138) comprises at least one spiral surface (160) about the rotation axis (136c, 138c) forming a pinch surface of the tubing (36, 38) so as to vary a fluid passage section through a tubing (36, 38) as the member (136, 138) rotates.

4. A cassette according to claim 3, wherein the movable members (136, 138) are mounted in recesses (134) of a plate (132) arranged inside the housing (90) and integral with the housing (90), each recess (134) receiving a movable member.

5. A cassette according to one of claims 2 to 4, wherein each movable member (136, 138) includes an end (136a, 138b) accessible from outside the housing (90) and provided with a first rotating coupling means (154) for cooperating with a second rotating coupling means of an actuator.

6. Cassette according to claim 4 or claims 4 and 5, **characterized in that** the movable parts are centred and rotatably guided at one end into an opening in the housing and at an opposite end into an opening in the plate.

7. A cassette according to one of claims 2 to 6, wherein the bag (100) has a substantially rectangular shape comprising a first flexible wall (122) and a second flexible wall substantially parallel to each other in both the empty and liquid-filled states.

8. A cassette according to one of claims 1 to 7, wherein the housing (90) has a shape substantially corresponding to that of a rectangular parallelepiped and comprises a lower half-shell (96) and an upper half-shell (98) fixed with each other, the lower half-shell (96) being rigid and the upper half-shell (98) being rigid or flexible.

9. A cassette according to claim 8, wherein the upper half shell (98) comprises an opening (108) or central recess whose shape and size are determined to allow the propagation of a wave of the liquid from the pocket (100) into the opening (108) when the cassette (16) containing a liquid is oscillated about a horizontal axis, the opening being disposed upward.

10. A cassette according to claim 8 or 9, wherein the conduits (36, 38) extend through one of the peripheral edges (102a, 102b) of one of the lower (96) or upper (98) half shells.

11. A cassette according to one of claims 1 to 10, comprising a conduit support strip (34), arranged outside the housing (90) and traversed by at least some of the conduits (36, 38).

12. A cell culture incubator, comprising a thermostatic enclosure (28) and a device for supporting and agitating (14) a cell culture cassette according to one of the above claims and means for positioning and locking the cell culture cassette (16) in a given position in the support device, the support and agitation device (14) comprising a tray (54) for supporting the cell culture cassette (16), said tray (54) carrying electromechanical actuators (82) for opening/closing the valves (128, 130), one outlet of which carries a second coupling means for cooperating with a first coupling means (154) of the valves (128, 130) of the cassette when the cassette (16) is in said given position.

13. An incubator according to claim 12, wherein the tray (54) is rotatably mounted around a horizontal axis (63) about which the tray (54) is intended to oscillate for agitating and homogenizing the contents of the bag (100).

14. Incubator according to claim 13, wherein the support and agitation device (14) comprises a connecting rod (64), one upper end of which is connected via an oscillator to the plate (54) and the lower end of which is connected to a crank (68) rotatably driven by the shaft of a motor whose axis (70) is substantially horizontal.

15. An incubator according to one of claims 12 to 14, comprising a door (22) for sealing an opening (26) of the enclosure (28), said opening (26) comprising a peripheral edge (24) in which a recess (32) is formed whose shape is adapted to receive the support strip (34) for the conduits of a cassette according to claim 15, the strip (34) forming a sealing member between the door (22) and the peripheral edge when the door is in the closed position of the enclosure (28).

16. A cell culture automated apparatus comprising at least one incubator (12) according to one of claims 12 to 14, and a computer control system including data capture and recording means for regulating the culture conditions in an enclosure of said at least one incubator and for controlling the valves of the cassette housed in the enclosure.

17. A method of cell culture by means of an automated apparatus according to claim 16, **characterized in that** it comprises the following steps:
a) placing a cell culture cassette (16) according to one of claims 1 to 11 inside the incubator enclosure (12) in a thawing position on the support and agitation device (14);
b) feeding the bag (100) with cells to be cultured;
c) placing the cassette (16) on the support and agitation device (14) in a cell culture position;
d) agitating the cassette (16) for a given period of time to homogenize its contents;
e) maintaining the cassette (16) under incubation conditions for several days; and
f) recover the contents of the cassette (16) by means of one of the tubes of the cassette (16).

18. A process according to claim 17, **characterized in that** it comprises:
- during step d), one or more sampling steps (196) for sampling the contents of the bag, each preceded by a step of tilting the support tray (54) from a horizontal culture position to an inclined position in which the sampling area of the bag (100) represents the lowest point of the bag (100).
